# FASCICULE DE BREVET EUROPEEN

(11) **EP 0 245 136 B1**
(45) Date de publication et mention de la délivrance du brevet: **23.03.1994**
(21) Numéro de dépôt: 87400790.9
(22) Date de dépôt: 08.04.1987
(51) Int. Cl.: C12N 15/00, C12N 15/62, C12N 7/00, C12P 19/34, C12P 21/02, C07K 15/00, C07K 15/14, A61K 39/21, G01N 33/569

(54) **Vecteur viral, codant pour une glycoprotéine du virus responsable du SIDA, vaccin et anticorps**
Für ein Glykoprotein des AIDS-Virus kodierende Virus-Vektoren, Vakzin und Antikörper
Viral vector encoding a glycoprotein of the AIDS virus, vaccine and antibodies

(30) Priorité: 08.04.1986 FR 8605043; 29.10.1986 FR 8615106
(43) Date de publication de la demande: 11.11.1987
(73) Titulaire: TRANSGENE S.A., 67082 Strasbourg Cédex (FR); INSTITUT PASTEUR, F-75015 Paris (FR)
(72) Inventeur: Kieny, Marie-Paule, F-67100 Strasbourg (FR); Rautmann, Guy, F-67000 Strasbourg (FR); Lecocq, Jean-Pierre, F-67116 Reichstett (FR); Wain Hobson, Simon, F-78180 Montigny-Le-Bretonneux (FR); Girard, Marc, F-75015 Paris (FR); Montagnier, Luc, F-92350 Le Plessis-Robinson (FR)
(74) Mandataire: Warcoin, Jacques

(56) Documents cités:
- BIO/TECHNOLOGY, vol. 4, no. 3, mars 1986, page 166, Nature Publishing Co.; H.BIALY: "IL-2 crystals, TNF tests, and AIDS vaccines"
- SCIENCE, vol. 227, 1 février 1985, pages 484-492; R. SANCHEZ-PESCADOR et al.:"Nucleotide sequence and expression of and AIDS-associated retrovirus (ARV-2)"
- NATURE, vol. 312, 8 novembre 1984, pages 163-166; M.P. KIENY et al.:"Expression of rabies virus glycoprotein from a recombinant vaccinia virus"
- SCIENCE, vol. 233, 11 juillet 1986, pages 209-212; L.A. LASKY et al.:"Neutralization of the AIDS retrovirus by antibodies to a recombinant envelopeglycoprotein"
- BIO/TECHNOLOGY, vol. 4, no. 9, septembre 1986, pages 790-795, New York, US; M.P. KIENY et al.: "AIDS virus ENV protein expressed from a recombinant vacciniavirus"
- NATURE, vol. 320, 10 avril 1986, pages 535-537; S. CHAKRABARTI et al.:"Expression of the HTLV-III envelope gene by a recombinant vaccinia virus"
- NATURE, vol. 320, 10 avril 1986, pages 537-540; S.L. HU et al.: "Expression ofAIDS virus envelope gene in recombinant vaccinia viruses"
- SCIENCE, vol. 233, 8 août 1986, pages 655-659; A.G. FISHER et al.: "Infectiousmutants of HTLV-III with changes in the 3'region and markedly reducedcytopathic effects"
- Science, vol.228, pp.1091-1094, 1985 (DV)
- Science, vol.228, pp.1094-1096, 1985 (DVI)

## Description

La présente invention concerne plus particulièrement un vaccin destiné à la prévention du S.I.D.A..

Le syndrome d'immunodéficience acquise (S.I.D.A.) est une affection virale qui présente maintenant une importance majeure en Amérique du Nord, en Europe et en Afrique centrale.

Les estimations récentes suggèrent que environ 1 Million d'Américains peuvent avoir été exposés au virus du S.I.D.A. Les individus affectés présentent une immunodépression sévère et la maladie est, en général, fatale.

La transmission de la maladie s'effectue le plus souvent par contact sexuel, bien que les personnes utilisant des stupéfiants par voie intraveineuse représentent également un groupe à haut risque ; d'autre part, un grand nombre d'individus ont été infectés par ce virus après avoir reçu du sang ou des produits sanguins contaminés.

L'agent causal de cette affection est un rétrovirus. De nombreuses affections animales ont été attribuées auxrétrovirus, mais c'est seulement récemment que des rétrovirus affectant des hommes ont pu être décrits.

Alors que des rétrovirus des cellules T humaines (HTLV: human T leukemia virus)de types I et II ont été impliqués comme agent causal de certaines leucémies des cellules T chez les adultes, le rétrovirus associé à des lymphadénopathies (virus LAV), qui est également appelé virus HTLV III ou A.I.D.S.-related virus (ARV), est maintenant couramment accepté comme l'agent responsable du S.I.D.A.

Le génome du rétrovirus LAV a été caractérisé de façon très complète (Wain-Hobson et al., 1985 ; Ratner et al., 1985 ; Muesing et al., 1985 ; Sanchez-Pescador et al., 1985) et des informations sur la séquence indiquent une relation étroite avec le groupe des lentivirus. Les lentivirus, dont le prototype est le virus ovin Visna, sont les agents de maladies à progression très lente et qui présentent typiquement une période d'incubation prolongée. Le LAV et le virus Visna partagent de nombreuses similarités, en particulier dans leur tropisme pour le tissu neural.

Par analogie avec d'autres rétrovirus bien connus, les trois plus importantes parties du génome du LAV ont été désignées par gag, pol et env. La séquence du gène env incluant la séquence de la gp12O et de la gp41 révèle des caractéristiques qui étaient attendues d'une glycoprotéine d'enveloppe transmembranaire et l'identité du précurseur de la protéine env, gp16O,constitué par la gp12O et la gp41, a été confirmée par un séquençage direct des acides aminés.

Dans ce qui suit, la gp41 sera parfois dénommée gp40 ou gp 42.

Des anticorps dressés contre la protéine env gp160 et ses produits de clivage gp120 et gp41, sont communément détectés dans le sérum de patients ayant le S.I.D.A., et la glycoprotéine env représente l'antigène de surface majeur du virus du S.I.D.A.

La protéine env est ainsi le candidat le plus prometteur pour développer une stratégie de vaccination, c'est pourquoi l'attention a été concentrée sur cette protéine et sur sa séquence codante.

Un grand nombre de groupes ont rapporté l'expression de la protéine env dans les bactéries. Toutefois, l'absence de glycosylation et de structuration post-traductionnelle peuvent compromettre le pouvoir immunogène des matériaux synthétisés par de tels micro-organismes.

C'est pourquoi la présente invention propose d'utiliser comme vecteur d'expression de la protéine env un vecteur viral permettant l'expression de la protéine dans un environnement qui permettra sa glycosylation et sa restructuration post-traductionnelle.

C'est pourquoi la présente invention concerne un vecteur viral caractérisé en ce qu'il comporte tout ou partie du gène env du virus responsable du S.I.D.A.

Parmi les vecteurs viraux utilisables, il faut citer plus particulièrement les poxvirus, et notamment le virus de la vaccine (VV).

Le virus de la vaccine est un virus à ADN double brin qui a été utilisé très largement dans le monde entier pour contrôler et éradiquer la variole. Des développements techniques récents ont permis le développement de ce virus comme vecteur de clonage et des virus recombinants vivants ont permis d'exprimer des antigènes étrangers et même d'obtenir des immunisations contre différentes maladies virales ou parasitaires.

Ainsi, plusieurs groupes ont récemment mis en évidence l'utilisation de recombinants de ce type pour exprimer l'antigène de l'Influenza, de l'hépatite B et la glycoprotéine de la rage pour immuniser contre ces maladies (Smith et al., 1983 ; Panicali et al., 1983 ; Kieny et al., 1984).

L'expression d'une séquence codant pour une protéine étrangère par le virus de la vaccine (VV) implique nécessairement deux étapes :
1) la séquence codante doit être alignée avec un promoteur de VV et être insérée dans un segment non essentiel de l'ADN de VV, cloné dans un plasmide bactérien approprié ;
2) les séquences d'ADN de VV situées de part et d'autre de la séquence codante doivent permettre des recombinaisons homologues in vivo entre le plasmide et le génome viral ; une double recombinaison réciproque conduit à un transfert de l'insert d'ADN du plasmide dans le génome viral dans lequel il est propagé et exprimé (Panicali et Paoletti, 1982 ; Mackett et al., 1982 ; Smith et al., 1983 ; Panicali et al., 1983).

Bien entendu, l'utilisation de ce type de vecteur implique souvent une délétion partielle du génome du virus vecteur.

La présente invention concerne plus particulièrement un vecteur viral caractérisé en ce qu'il comporte au moins :
- une partie du génome d'un virus vecteur,
- un gène codant pour l'une des glycoprotéines (gp) de l'enveloppe du virus responsable du SIDA,
- ainsi que les éléments assurant l'expression de cette glycoprotéine dans des cellules.

L'invention concerne également les ADN recombinants correspondant auxdits vecteurs viraux.

Il convient de remarquer que les glycoprotéines (gp) de l'enveloppe du virus responsable du SIDA sont au nombre de 3, désignées par leur masse en kDA, à savoir la gp16O, la gp12O et la gp41 ; la première, gp16O, est en fait le précurseur des deux dernières protéines. Ces dénominations ne sont pas encore figées et la gp41 est parfois appelée gp4O ou gp42 mais les différences de masse font que ces 3 glycoprotéines sont parfaitement identifiables, quelle que soit leur dénomination.

Par virus responsable du SIDA, on entend notamment désigner le virus LAV, le virus HTLV III ou ARV, de même que d'éventuels mutants ponctuels ou des délétions partielles de ces virus, ainsi que les virus apparentés.

Les vecteurs viraux, dans la partie correspondant au génome du virus vecteur (distinct du virus responsable du S.I.D.A.), peuvent être constitués à partir du génome d'un virus d'origine quelconque. Toutefois, on préfèrera utiliser une partie du génome d'un poxvirus et plus particulièrement une partie du génome de la vaccine.

Les conditions nécessaires pour l'expression d'une protéine hétérologue dans le virus de la vaccine ont été rappelées précédemment.

De façon générale, le gène en cause, par exemple le gène env, devra, pour pouvoir être exprimé, être sous la dépendance d'un promoteur d'un gène de la vaccine, ce promoteur sera en général le promoteur de la protéine 7,5K de la vaccine. En outre, la séquence codante devra être clonée dans un gène non essentiel de la vaccine qui pourra éventuellement servir de gène marqueur.Dans la plupart des cas, il s'agira du gène TK.

Parmi les glycoprotéines de l'enveloppe que l'on souhaite voir exprimer, il faut citer les trois protéines mentionnées précédemment, à savoir la gp 160, la gp 41 et la gp 120.

De façon générale, on préfèrera faire exprimer le gène d'enveloppe complète, c'est-à-dire le gène env comportant la séquence signal et la séquence transmembranaire de ce gène.

Les premiers essais conduits avec un vecteur viral dans lequel a été cloné le gène codant pour la protéine env totale a conduit à proposer des modifications de ce gène pour améliorer l'immunogénicité des produits d'expression.
On a constaté un relargage important de la protéine env dans les surnageants de culture (relargage qui se produit, probablement in vivo, dans les liquides circulants). Ceci peut être dû à un mauvais accrochage de la protéine dans la membrane cellulaire ; on sait en outre que la présentation des antigènes à la surface des cellules est très importante pour l'induction d'une réponse immunitaire avec le système vaccine. On propose donc de modifier le gène env de façon à améliorer l'ancrage de la glycoprotéine dans la membrane cellulaire.

Pour ce faire, le gène env pourra être modifié au niveau de sa partie codant pour la zone transmembranaire afin de remplacer le codon correspondant à une arginine par un codon correspondant à une isoleucine.

Il existe également une possibilité d'améliorer l'ancrage en remplaçant et/ou ajoutant à la zone transmembranaire de la protéine env la zone transmembranaire d'un virus hétérologue, par exemple la zone transmembranaire de la gp du virus de la rage.

De plus, il se pourrait que la protéine ne soit pas bien assemblée à la suite de son expression^{.} En effet, le peptide signal est assez atypique, et pourrait nuire à l'exportation complète de la protéine. C'est pourquoi il est proposé de remplacer et/ou d'ajouter une séquence signal qui provienne d'un virus hétérologue, par exemple la séquence signal de la gp du virus de la rage.

Enfin, il semble que c'est la gp 120, plutôt que la gp 160, qui est relarguée par les cellules. Elle peut, d'une part, fournir un leurre pour le système immunitaire, d'autre part en accord avec des données récentes, aller se fixer sur les cellules T4, ce qui pourrait avoir pour effet d'inactiver les cellules T4 ou de les faire apparaître comme étrangères aux autres cellules T.

Il peut donc être intéressant d'obtenir une protéine env gp 120 qui ne puisse pas être relarguée. Ceci est effectué en modifiant le gène env entre les séquences codantes de la gp 120 et de la gp 41 pour supprimer les sites de clivage par les protéases situé entre la gp 120 et la gp 41 en particulier par la suppression du site REKR.

Dans les plasmides selon l'invention, on effectue au moins une mutation supplémentaire dans un site correspondant à une séquence KRR située 8 aminoacides en aval de la séquence REKR. La gpl60 ainsi obtenue n'est plus clivée.

Les vecteurs selon la présente invention comportent également une séquence codant pour la gp41 dépourvue de la séquence correspondant à son peptide N-terminal hydrophobe dont on pense qu'il pourrait être responsable du pouvoir syncitial de la protéine env c'est-à-dire la capacité pour le virus de faire fusionner les cellules et d'obtenir une cellule géante ou syncitium.

Enfin, la présente invention concerne des vecteurs viraux dans lesquels les régions extracytoplasmique et intracytoplasmique de la gpl60 sont fusionnées en phase après délétion du peptide hydrophobe C-terminal ce qui permet d'obtenir une sécrétion de la glycoprotéine.

De façon générale, les vecteurs viraux selon l'invention comportent une séquence codant pour l'une des protéines suivantes : 
J
- S - gp120 ———————— gp40 - (tm) -
. S est un peptide signal,
. gpl20 est la glycoprotéine l20,
. J schématise la partie de jonction entre la gpl20 et la gp40 dépourvue de site de clivage par les protéases,
. gp40 est la glycoprotéine 40,
. tm est un peptide transmembranaire
ou bien encore pour une protéine de structure :
- S - gp40 - tm
ou
- S- gpl20 - tm
Le peptide signal et la séquence transmembranaire peuvent être ceux du virus responsable du SIDA ou bien être hétérologues, en particulier provenir du virus de la rage ou du VSV ou de tout virus à enveloppe.

La gp40 peut éventuellement être dépourvue de son extrémité N-terminale hydrophobe.

La première invention concerne principalement l'utilisation de vecteurs viraux pour l'obtention des glycoprotéines codées par le gène env du virus LAV dans des cultures cellulaires. Il s'agit donc dans un premier temps de cellules de mammifères qui ont été infectées par un vecteur viral selon l'invention ou bien qui peuvent contenir l'ADN recombinant correspondant ; parmi ces cellules, il faut citer plus particulièrement les cellules diploïdes humaines, des cultures primaires ainsi que les cellules Vero. Bien entendu, il est possible de prévoir d'autres types de cellules comme cela ressortira d'ailleurs des exemples ci-après.

Les glycoprotéines ainsi obtenues peuvent être utilisées après purification pour la réalisation de vaccins.

Il est également possible de prévoir l'utilisation directe des vecteurs viraux selon l'invention afin d'effectuer une vaccination, les glycoprotéines étant alors produites in situ et in vivo.

Il est intéressant de prévoir l'utilisation associée de plusieurs agents vaccinants administrés conjointement ou séparément, en particulier les agents vaccinants correspondant aux vecteurs exprimant séparément la gpl20 et la gp40 ayant subi la modification décrite précédemment. Par exemple, il peut être intéressant d'utiliser conjointement les agents vaccinants issus des vecteurs ll36 et ll38qui seront décrits ci-après.

Enfin, la présente invention concerne également les anticorps dressés contre les glycoprotéines précédentes obtenus par infection d'un organisme vivant avec un vecteur viral tel que décrit précédemment et récupération des anticorps induits après un temps déterminé.

Les techniques mises en oeuvre pour l'obtention des glycoprotéines, les cultures cellulaires et les techniques de vaccination sont identiques à celles qui sont pratiquées actuellement avec les vaccins connus et ne seront pas décrites en détail.

La présente invention sera mieux comprise à la lecture des méthodes et exemples suivants.

Cinq figures illustrent les exemples :
- la figure 1 représente l'action de l'endo-F sur les protéines synthétisées par les recombinants VVTGeLAV9-1 et VVTGeLAV1132 et immunoprécipitées grâce à un sérum anti-LAV. Dans cette figure, les poids moléculaires sont donnés en kilodaltons, et on représente par :
   . P, le culot cellulaire
   . S, le surnageant
   . u, les produits obtenus sans traitement
   . e, les produits obtenus après traitement à l'endo-F.
- La figure 2 représente la reconnaissance des protéines du virus LAV par les sérums de souris vaccinées avec le recombinant VVTGeLAV9-1. Dans cette figure, T représente les cas où le sérum utilisé est celui d'un malade atteint de SIDA. Les poids moléculaires sont exprimés en kdaltons.
- La figure 3 représente l'immunoprécipitation des protéines synthétisées par les virus de la vaccine recombinants portant le gène env. Dans cette figure, les poids moléculaires sont en kDaltons.

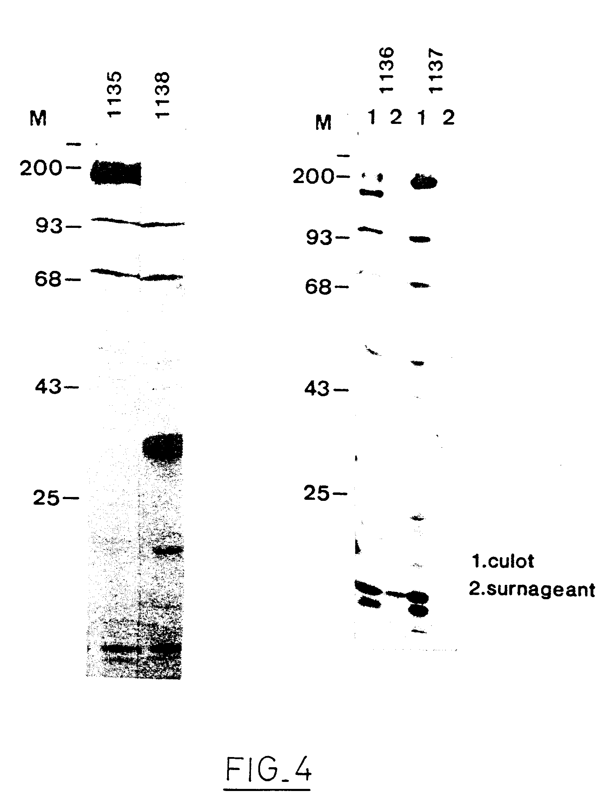

- la figure 4 représente une immunoprécipitation des protéines synthétisées par les virus recombinants VV.TG.eLAV ll35, ll36, ll37 et ll38.
   Le virus ll35 synthétise une gpl60 qui n'apparait pas dans le surnageant de culture.
   En ce qui concerne les virus ll36 et ll38, ils produisent des protéines gpl20 et gp40 respectivement, associées au culot cellulaire.
   Le virus ll37 produit une protéine légèrement plus petite que le virus ll35, avec un PM en accord avec celui attendu.

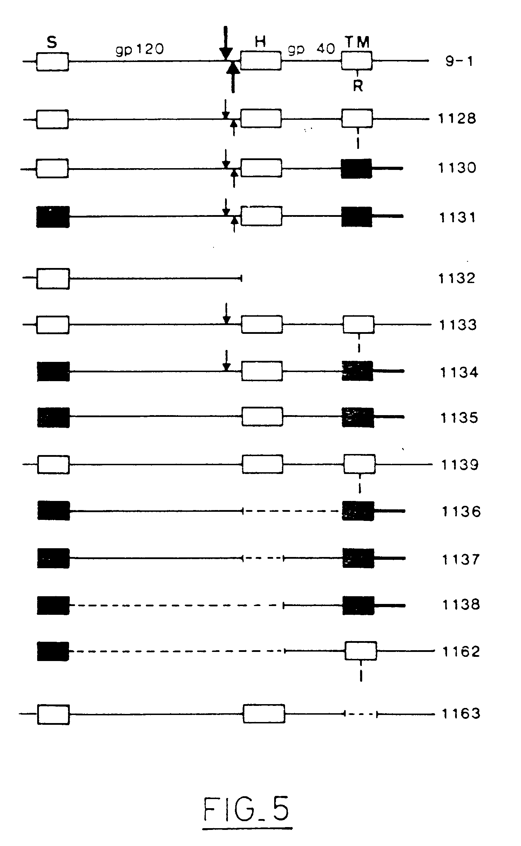

- la figure 5 représente la structure des protéines env synthétisées par les virus recombinants.
   - S :: peptide signal
   - H :: zone hydrophobe interne
   - TM :: zone d'ancrage transmembranaire
   - ↑ :: site de clivage gpl20/gp40
   - ■ :: séquence provenant de la glycoprotéine rabique

### METHODES

Clonages : Maniatis et al., 1982.

Enzymes : utilisées selon les prescriptions du fournisseur.

Mutagénèse localisée : méthode dérivée de Zoller and Smith, 1983.

Transfert dans la vaccine : Kieny et al., 1984.

Seule différence : les cellules humaines 143B remplacent les cellules LMTK⁻.

### Préparation du stock de virus

Les cellules primaires de poulet "germ free" sont infectées à 0,01 pfu/cellule pendant 4 jours à une température de 37°C (milieu MEM + 5 % NCS).

### Purification du virus

On effectue une centrifugation du stock de virus ci-dessus pendant 15 minutes à 2 500 tours (Rotor GSA Sorvall). Le surnageant est mis de côté. On reprend le culot dans un tampon RSB (Tris HCl 10 mM pH 7,4, KCl 10 mM, MgCl₂ 1 mM) pendant 15 minutes à 4°C. On effectue un broyage au potter, puis une centrifugation pendant 15 minutes à 2 500 tours. Le surnageant est ajouté au précédent puis on effectue un deuxième broyage de la même façon.

Tous les surnageants sont déposés sur 10 ml de coussin de saccharose 36 % (p/v) (Tris 10 mM pH 8). On effectue une centrifugation pendant 2 heures à 14 000 tours (Rotor SW28, Beckman).

Le culot est repris, dissocié et remis sur un deuxième coussin identique. Le 2ème culot est repris dans 5 ml PBS et chargé sur un gradient 20-40 % de Saccharose (Tris 10 mM pH 8) (même rotor). On effectue une centrifugation pendant 45 minutes à 12 000 tours.

On récupère la bande de virus - Elle est culottée par centrifugation pendant 1 heure à 20 000 tours. Le culot est repris dans du Tris 10 mM pH 8.

### Immunoprécipitations

On effectue une infection de cellules BHK-21 (boîtes de 3 cm de diamètre, 10⁶ cellules par boîte, cultivées en G-MEM + 10 % FCS) à 0,2 pfu/cellule pendant 18 heures. Le milieu est décanté et remplacé par 1 ml de milieu sans méthionine et 10 µl de méthionine ³⁵S (Amersham) par boîte.

On ajoute un excès de méthionine non radioactive après 2 heures.

A la fin du marquage, on effectue un grattage des cellules infectées, une centrifugation pendant 1 minute dans une centrifugeuse Eppendorf, une séparation des fractions surnageant et culot, un lavage du culot une fois en tampon PBS, puis une immunoprécipitation et un gel d'électrophorèse (selon Lathe et al., 1980).

### Traitement endo-F

Après immunoprécipitation des protéines marquées par un sérum de malade atteint du SIDA, la fraction protéine-A sepharose est reprise dans :
0,2 M phosphate de Na, pH 6,1
0,05 % SDS
0,1 % Nonidet P40
0,1 % Beta-mercaptoéthanol
0,1 % EDTA pH 8
et bouillie pendant 5 minutes pour dénaturer les protéines.

On effectue une incubation pendant 20 heures à 37°C, avec 4 unités d'Endo-F par ml, puis une précipitation pendant 2 minutes dans la glace avec 1/5 de volume de TCA 100 %. On lave le culot 3 fois à l'acétone 80 %, on ajoute le tampon d'échantillon et on charge sur gel SDS.

### Dosage des anticorps par test ELISA

- LAV
   Utilisation du test ELAVIA (Pasteur-Diagnostic) avec un deuxième anticorps mouton-antisouris lié à la peroxydase.
- Vaccine
   Des plaques 96 trous (NUNC) à fond plat sont incubées pendant 18 heures à 37°C avec 10⁷ pfu de virus de la vaccine type sauvage en tampon carbonate. Les plaques sont ensuite saturées avec de la gélatine 0,01 %. Les sérums de souris sont alors adsorbés sur les plaques et on effectue le reste du protocole comme pour l'Elisa LAV.

Lectures à 492 nM.

### EXEMPLE 1

### Construction des plasmides hybrides

Les tailles combinées des différents éléments nécessaires pour letransfert de la séquence codant pour le gène env dans le génome de VV et son expression subséquente sont de l'ordre de plusieurs Kb. Il a donc été jugé nécessaire de minimiser la taille du plasmide de réplication dans E. coli utilisé pour le travail de construction de façon à faciliter les manipulations nécessaires.

Le fragment HindIII (Hin-J) du génome de VV contient le gène complet de la thymidine kinase (TK) qui a déjà été utilisé précédemment pour permettre l'échange et la recombinaison de l'ADN inséré dans le génome de VV (Mackett et al., 1982). Il est important de noter que le transfert d'un insert dans le gène TK du génome de VV crée un virus TK déficient qui peut être sélectionné. Il a tout d'abord été nécessaire de produire un plasmide de petite taille portant un site unique HindIII utilisable pour l'intégration du fragment Hin-J VV. En outre, il était nécessaire d'éliminer les séquences de restriction non nécessaires du plasmide de façon à permettre les manipulations suivantes.

La construction a été amorcée à partir du plasmide pML2 (Lusky et Botchan, 1981) qui est un vecteur dérivé du plasmide pBR322 par délétion spontanée dans lequel le segment entre les nucléotides 1089 et 2491 a été perdu. D'abord la séquence de PstI a été éliminée par insertion du fragment AhaIII-AhaIII de pUC8 (Vieira et Messing, 1982) entre deux sites AhaIII de pML2 en éliminant 19 paires de bases. On a utilisé la méthode du "linker-tailing" (Lathe et al., 1984) pour insérer un linker HindIII entre lessites NruI et EcoRI traité par S1 de ce plasmide, en éliminant le site BamHI. Ceci conduit à un plasmide de 2049 paires de bases portant le gène bêta-lactamase fonctionnel (conférant la résistance à l'ampicilline) et comportant en outre une origine de réplication active dans E. coli et un site de restriction unique HindIII.

Cette construction a été appelée pTG1H.

Le fragment Hin-J de l'ADN de VV portant le gène TK a préalablement été cloné dans un vecteur provenant de pBR327 (Drillien et Spehner, 1983). Ce fragment de 4,6 Kb a été recloné dans le site HindIII de pTG1H. Un clone a été sélectionné dans lequel le gène TK est situé distalement par rapport au gène codant pour la résistance à l'ampicilline.

Cette construction pTG1H-TK a été utilisée comme vecteur dans l'expérience suivante.

L'étape suivante a été d'isoler un promoteur de VV utilisable pour commander l'expression de la séquence codant pour le gène à exprimer. Le promoteur d'un gène précoce codant pour une protéine de 7500 daltons (7,5 K) a déjà été utilisé avec succès dans un but identique (Smith et al., 1983) et on a donc procédé à l'isolement de ce segment.

Le gène 7,5 K est situé sur l'un des plus petits fragments SalI (fragment Sal-S) du génome de VV type WR (Venkatasan et al., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est transféré sur le bactériophage vecteur M13mp701 (voir Kieny et al., 1983), par digestion SalI et religation, en conduisant ainsi au phage M13TGSal-S.

Dans ce clone, un site ScaI se trouve immédiatement à proximité de l'ATG d'initiation du gène 7,5 K. En aval du gène 7,5 K se trouvent situés des sites uniques BamHI et EcoRI provenant du vecteur. Les sites BamHI et ScaI sont fusionnés par l'intermédiaire d'un linker BglII 5' -CAGATCTG-3' après avoir complété les extrémités générées par digestion BamHI avec le fragment Klenow de la polymérase de E. coli. Ce procédé élimine le site ScaI mais reconstitue le site BamHI et déplace le site unique EcoRI en aval. En même temps, le site SalI (AccI) en aval est éliminé, le site Sal I en amont devient donc unique.

Cette construction est appelée M13TG 7,5 K.

A l'intérieur du fragment Hind-J de l'ADN de VV se trouvent situés des sites ClaI et EcoRI qui sont séparés par environ 30 paires de bases (Weir et Moss, 1983). Le fragment promoteur de 7,5 K présent dans M13TG7, 5K est excisé par AccI et EcoRI et cloné entre les sites ClaI et EcoRI de pTG1H-TK pour générer pTG1H-TK-P7,5K.

Cette construction conduit au transfert des sites BamHI et EcoRI uniques du vecteur M13 immédiatement en aval de la séquence du promoteur 7,5K. Ces sites uniques BamHI et EcoRI sont utilisés dans la construction suivante.

Le segment polylinker du bactériophage M13TG131 (Kieny et al., 1983) est excisé par EcoRI et Bgl II et inséré entre les sites EcoRI et BamHI du plasmide pTG1H-TK-P7,5K, générant pTG186-poly. Dans cette construction, 10 sites de restriction sont disponibles pour le clonage d'un gène étranger sous le contrôle de P7,5K.

### EXEMPLE 2

### Construction du plasmide portant la séquence env.

Afin d'obtenir une séquence codante pour env, on effectue tout d'abord l'assemblage des deux segments proviraux clonés dans les plasmides PJ19-6 et PJ19-13.

De façon à assurer une traduction adéquate du mARN de env, la séquence de nucléotides autour du site d'initiation de la traduction présumée du gène env a été modifiée pour s'adapter à la séquence consensus des gènes eucaryotes et ceci par une mutagénèse dirigée avec un oligonucléotide au voisinage de la position 5767.

Les plasmides PJ19-13 et PJ 19-6 contiennent des fragments HindIII du génome proviral de LAV comprenant les nucléotides 1258 à 1698 et 1698 à 9173 respectivement.

Un fragment EcoRI-Kpn1 de PJ19-13 (contenant l'ATG d'initiation de env) a été inséré dans le phage M13TG130 et on a effectué une mutagénèse dirigée avec un oligonucléotide (séquence 5'CTCTCATTGTCACTGCAGTCTGCTCTTTC), pour introduire un site PstI en amont du codon d'initiation de la traduction de env (position 5767) et afin de substituer le G à la position -3 par un A. Le fragment muté a été introduit ensuite entre les sites EcoRI et KpnI du plasmide pTG1-POLY (qui est un mini plasmide de 2,1 kb similaire à pTG1H mais qui contient un segment polylinker de M13TG131.

Le fragment KpnI-HindIII provenant de PJ 19-13 a été ensuite cloné dans le même plasmide (entre KpnI et HindIII), suivi par un fragment HindIII-XhoI de PJ 19-6 (entre HindIII et SalI) pour générer une séquence codante env complète flanquée par deux sites PstI (plasmide pTG1124).

L'introduction de ces deux sites de restriction PstI permet une manipulation plus aisée de l'ADN du gène env dans la suite de la construction. Comme cela a été indiqué précédemment, l'expression d'une protéine hétérologue dans le virus de la vaccine nécessite que la séquence codante soit alignée avec une séquence de promoteur de la vaccine et soit insérée dans un segment non essentiel de l'ADN de la vaccine. Cet ADN situé de part et d'autre permet la recombinaison avec le génome de la vaccine in vivo par une double recombinaison réciproque, qui transfère la séquence codante et le promoteur accompagnant dans le génome de la vaccine.

Pour ce faire, le fragment PstI-PstI mentionné précédemment a été cloné dans le site PstI de pTG186-POLY. On obtient ainsi un plasmide dénommé pTG1125.

Le plasmide pTG186-POLY peut être généré à partir du plasmide pTG188 digéré par PstI et religué par la ligase T4.

Le plasmide pTG188 a été déposé le 20 juin 1985 à la Collection Nationale de Cultures de Microorganismes de l'Institut Pasteur - 28, rue du Docteur Roux - 75015 PARIS sous le numéro suivant :
E. Coli 5K pTG188 = n^{o} I 458.

Le transfert de la séquence codante du gène env et du promoteur accompagnant dans le génome de la vaccine est effectué comme suit.

### EXEMPLE 3

### Clonage dans le virus de la vaccine pour générer VV.TG.e LAV 9-1

La stratégie décrite par Smith et coll. (1983) repose sur l'échange in vivo entre un plasmide portant un insert dans le gène VV TK et le génome viral de type sauvage de façon à inactiver le gène TK porté par le virus. Les virus TK⁻ peuvent être sélectionnés par étalement sur une lignée cellulaire (TK-négative) en présence de 5-bromodéoxyuridine (5BUDR) (Mackett et coll., 1982). La thymidine kinase phosphoryle le 5BUDR en 5'-monophosphate, qui est ensuite converti en triphosphate. Ce composé est un analogue de dTTP et son incorporation dans l'ADN bloque le développement correct du virus. Un virus TK⁻ peut néanmoins répliquer son ADN normalement et il conduit à des plages virales visibles dans une lignée cellulaire également TK⁻.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression de son génome. L'ADN de VV purifié est non infectieux.

Afin de générer les recombinants, il est nécessaire d'effectuer simultanément l'infection cellulaire avec du virion VV et une transfection avec le segment d'ADN cloné qui présente de l'intérêt. Toutefois, la génération des recombinants est limitée à la petite proportion des cellules qui sont compétentes pour la transfection par l'ADN. C'est pour cette raison qu'il a été nécessaire de mettre en oeuvre une stratégie de "congruence" indirecte pour réduire le bruit de fond des virus parentaux non-recombinants. Ceci a été effectué en utilisant comme virus infectieux vivant un mutant thermosensible (ts) de la vaccine qui n'est pas capable de se propager à une température non permissive de 39,5°C (Drillien et Spehner, 1983). Lorsque les cellules sont infectées par un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu ; aucun virus ne se multipliera dans les autres cellules, en dépit du fait qu'elles ont été infectées. Si un plasmide recombinant contenant un fragment de l'ADN de vaccine tel que pTG1125 est inclus dans le mélange de transfection, à la concentration appropriée, avec l'ADN du type sauvage, il est également possible d'obtenir qu'il participe à la recombinaison homologue avec l'ADN de la vaccine dans les cellules compétentes.

Des monocouches de cellules primaires de fibroblastes d'embryons de poulets (CEF) sont infectées à 33°C avec VV-Copenhague ts7 (0,1 pfu/cellule) et transfectées avec un coprécipité au phosphate de calcium de l'ADN du virus de type sauvage VV-Copenhague (50 ng/10⁶ cellules) et le plasmide recombinant (50 ng/10⁶ cellules).

Après incubation pendant 2 heures à une température qui ne permet pas le développement du virus ts (39,5°C), les cellules sont incubées de nouveau pendant 48 heures à 39,5°C. Des dilutions de virus ts⁺ sont utilisées pour réinfecter une monocouche de cellules humaines 143 B à 37°C qui sont ensuite incubées en présence de 5BUDR (150 µg/ml). Différentes plaques de virus TK⁻ sont obtenues à partir de ces cellules qui ont reçu le plasmide recombinant, tandis que les cultures contrôles sans plasmide ne montrent pas de plaques visibles. Les virus TK⁻ sont ensuite sous-clonés par une deuxième sélection en présence de 5BUDR.

Une double recombinaison réciproque correcte entre le plasmide hybride pTG1125 et le génome de VV aboutit à l'échange du gène TK portant l'insert avec le gène TK du virus, les recombinants devenant ainsi TK⁻.

Les ADN purifiés à partir des différents virus recombinants TK⁻ sont digérés par HindIII et soumis à une électrophorèse sur gel d'agarose. Les fragments d'ADN sont transférés sur un filtre de nitrocellulose selon la technique décrite par Southern (1975). Le filtre est ensuite hybridé avec le plasmide pTG1125 nick-translaté au ³²P. Après lavage du filtre, ce dernier est fluorographié et des bandes de 3.85, 2.9, 0.8 Kb sont visibles sur l'autoradiographie quand le virus vaccine a incorporé le gène env du LAV. L'un de ces recombinants VV.TG. eLAV 9-1 a été sélectionné pour les études suivantes.

### EXEMPLE 4

### Protéine env synthétisée à partir d'un virus recombinant vaccine-LAV

Pour mettre en évidence l'expression du gène env du LAV à partir du virus vaccine hybride, on infecte des cellules de rongeur, BHK21, qui sont cultivées dans un milieu G-MEM + 10 % de sérum de veau foetal avec ledit recombinant VV.TG. eLAV 9-1.

Une monocouche semi-confluente fraîche (10⁶ cellules) est infectée avec 0,2 pfu/cellule et incubée pendant 18 heures.

Le milieu est ensuite éliminé et on ajoute un milieu à faible teneur en méthionine (1 ml pour 10⁶ cellules) supplémenté avec 10 µl/ml de méthionine ³⁵S. Les cellules sont incubées à 37°C et les protéines marquées sont collectées par centrifugation. Après séparation en culot et surnageant, les protéines sont incubées avec un sérum appartenant à un patient atteint de SIDA. Les protéines réagissant avec le sérum sont récupérées par adsorption sur une résine protéine A-sépharose et étalées par électrophorèse sur un gel de polyacrylamide SDS et autoradiographiées selon une technique décrite par Lathe et al., 1980. Les autoradiographies montrent que le sérum du patient atteint du SIDA lie spécifiquement trois protéines des extraits cellulaires infectés (le résultat est identique ou similaire à celui obtenu avec d'autres sérums de patients). Les poids moléculaires apparents de 160, 120 et 41 Kd suggèrent l'équivalence avec les bandes gp 160, gp 120 et gp 41 identifiées par des sérums de malades atteints du SIDA, dans une préparation de glycoprotéine env. authentique et dans des extraits de cellules infectées par le virus LAV. Cette observation que trois protéines sont exprimées à partir du vecteur recombinant portant seulement la séquence codant pour le gène env du LAV supporte l'hypothèse que la gp 120 et gp 41 sont générées par clivage protéolytique du produit de traduction primaire gp 160.

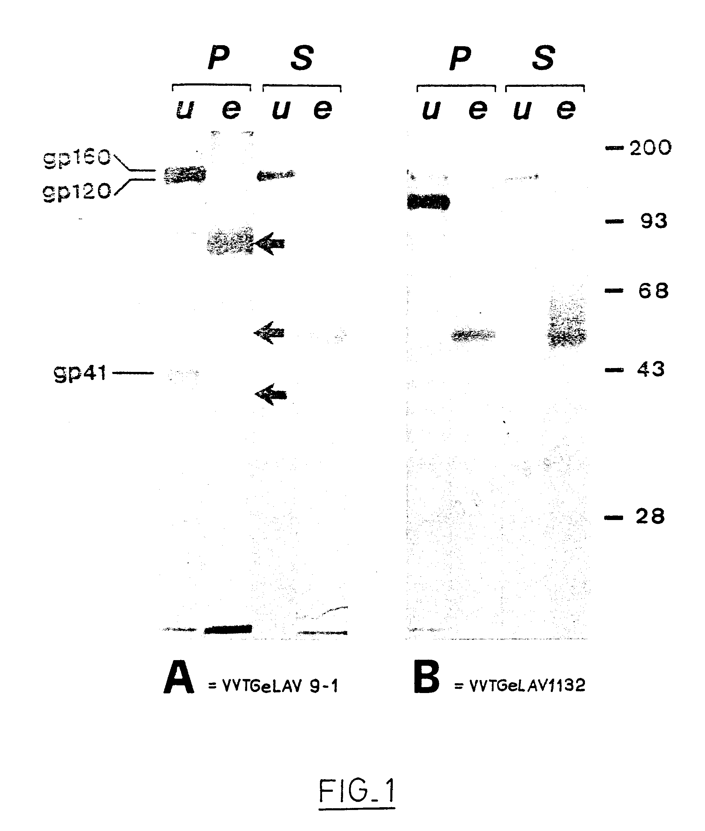

La séquence codant pour env conduit à un produit de traduction primaire d'environ 90 kDA alors que le précurseur de env obtenu par le procédé précédent présente un poids moléculaire apparent d'environ 160 kDA. Cette différence est attribuée à une glycosylation très importante. Par digestion avec l'endoglycosydase F qui élimine les groupes glycosyl, on a pu mettre en évidence une bonne corrélation entre les produits obtenus par la présente invention et les produits prévus (figure 1).

### EXEMPLE 5

### Mise en évidence des anticorps anti-env chez des souris vaccinées avec le virus VV.TG.e LAV 9-1

Des souris Balb/c mâles de 5 semaines sont vaccinées par injection sous-cutanée de 5.10⁷ pfu de virus VV.TG.e LAV 9-1 par animal. Elles reçoivent une injection de rappel avec la même dose après 2 semaines, et subissent une prise de sang 1, 2 et 4 semaines après le rappel. La présence d'anticorps dirigés contre des déterminants du virus LAV et du virus de la vaccine dans leurs sérums est recherchée.

Tous les animaux vaccinés donnent des sérums capables de réagir avec le virus de la vaccine dans un test ELISA. Par contre, la réponse en test ELISA contre le virus LAV est faible et peu reproductible. Pour améliorer la sensibilité des tests, une technique de "Western blot" a été utilisée. Cette méthode permet de mettre en évidence les anticorps capables de réagir avec les protéines du virus LAV après que celles-ci aient été dénaturées au SDS dans un gel d'électrophorèse et transférées sur une membrane de nitrocellulose. Dans cette expérience, les membranes de nitrocellulose employées sont celles du kit LAV-BLOT vendu par Diagnostic Pasteur et sur lesquelles les protéines du virus LAV sont déjà fixées. Ces membranes sont découpées en bandes, et chaque bande est incubée avec le sérum des souris vaccinées (dilution au 1/20). Un deuxième anticorps (mouton antisouris) lié à la peroxydase permet de visualiser les protéines du virus LAV qui ont fixé des anticorps de souris.

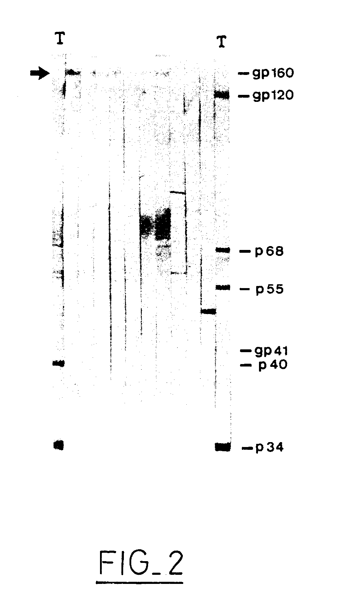

Plusieurs sérums (12/27) donnent une réaction spécifique avec une protéine de poids moléculaire autour de 160 kDA, correspondant à la gp 160 de env (figure 2). Dans un certain nombre de sérums, on observe également une réaction avec la protéine gp 41. Il faut noter que les sérums de quelques souris produisent en Western blot des signaux correspondant à des protéines non-identifiées de la préparation de virus LAV fixée sur les membranes.

### EXEMPLE 6

### Construction de pTG1128

Ce plasmide pTG1128 est identique au plasmide 1125 à ceci près que la séquence codant pour la zone transmembranaire a été mutée pour remplacer l'arginine par une isoleucine, ceci afin d'améliorer l'accrochage de la protéine dans la membrane cellulaire.

Le fragment HindIII-BamHI de pTG1124 contenant la zone transmembranaire de env décrit à l'exemple 2 est inséré dans le phage M13 TG131 après une digestion HindIII-BamHI. On obtient ainsi un phage M13 TG154.

On effectue ensuite sur ce phage M13 TG154 une mutagénèse localisée destinée à remplacer le codon codant pour l'arginine par un codon codant pour l'isoleucine. On utilise pour ce faire l'oligonucléotide suivant :
5' GGTTTAATAATAGTTTT 3'.

On obtient ainsi le phage M13 TG155, les séquences ayant été modifiées comme suit : 
Gly Leu Arg Ile Val
Séquence originale : GGT TTA AGA ATA GTT
Séquence mutée : GGT TTA ATA ATA GTT
Ile
Le fragment BamHI-HindIII ainsi muté est transféré de M13 TG155 dans le plasmide pTG1124 dans des sites équivalents pour donner le plasmide pTG1127 qui reconstitue le gène env comme précédemment sauf que le codon de l'arginine a été remplacé par un codon isoleucine.

Comme cela a été décrit dans l'exemple 1, le fragment PstI-PstI de pTG1127 est cloné dans le site PstI du plasmide pTG186-POLY pour donner le plasmide pTG1128.

### EXEMPLE 7

### Construction du plasmide pTG1130

Dans ce plasmide, la séquence codant pour la zone transmembranaire de la glycoprotéine rabique est fusionnée avec le début de la séquence codant pour la partie hydrophobe de la glycoprotéine env.

La zone transmembranaire de la glycoprotéine rabique provient d'un fragment BamHI-PstI du phage M13 TGRG151.

Ce fragment est cloné dans le phage M13 TG154 entre les sites BamHI et PstI (voir exemple précédent). On obtient ainsi le phage M13 TG156.

On effectue ensuite une mutagénèse localisée sur M13 TG156 pour fusionner en phase les séquences env et rage avec un oligonucléotide en formant une boucle 5' GCTGTGGTATATAAAATATGTATTACTGAGTG 3' 
Tyr Leu Lys Ile Phe Gly Lys Tyr Val
TAT ATA AAA ATA TTC ------ GGG AAG TAT GTA
tm env tm rage
On obtient ainsi le phage M13 TG157.

La zone transmembranaire (tm) de la glycoprotéine rabique qui vient d'être fusionnée avec le gène env est ensuite transférée dans le plasmide pTG1124.

Pour ce faire, on clone le fragment HindIII-BglII de M13 TG157 dans pTG1124 dont on a effectué une restriction HindIII-BamHI (ceci détruit les sites BamHI et BglII).

Le site BglII de M13 TG157 provient du fragment gp rage : 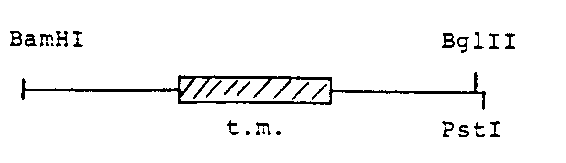
BamHI
BglII
t.m.
PstI
On obtient ainsi le plasmide pTG1126.

Comme précédemment le fragment PstI-PstI de pTG1126 est cloné dans le site PstI de pTG 186-POLY pour donner le plasmide pTG1130.

### EXEMPLE 8

### Construction de pTG1131

L'objectif de la construction de ce plasmide est de fusionner la séquence signal du gène env et la séquence signal de la glycoprotéine rabique.

La séquence signal de la glycoprotéine de la rage est prélevée à partir du plasmide pTG155 PRO sous forme d'un fragment BglII-HindIII qui est cloné dans les sites PstI-HindIII de M13 TG130 grâce à un adaptateur simple brin ayant la séquence suivante :
5' GATCTGCA 3'
On obtient ainsi le phage M13 TG158.

Le transfert du peptide signal de env dans M13 TG158 est ensuite réalisé afin de fusionner ce dernier avec le gène codant pour le peptide signal de la glycoprotéine rabique.

On effectue pour ce faire le clonage du fragment PstI traité à la nucléase S1 puis à la Klenow et KpnI dans M13 TG158 coupé par HindIII traité à la Klenow-KpnI : 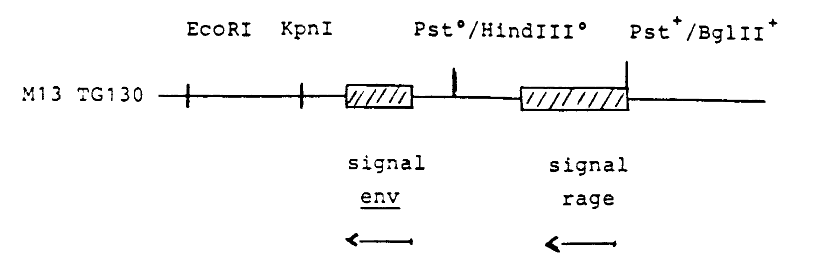
EcoRI   KpnI   Pst°/HindIII°   Pst+/BglII+
M13 TG130
signal   signal
env   rage
On obtient ainsi le plasmide M13 TG159.

On transfère le bloc KpnI-PstI de M13 TG159 dans M13 TG131 pour obtenir le plasmide M13 TG160. 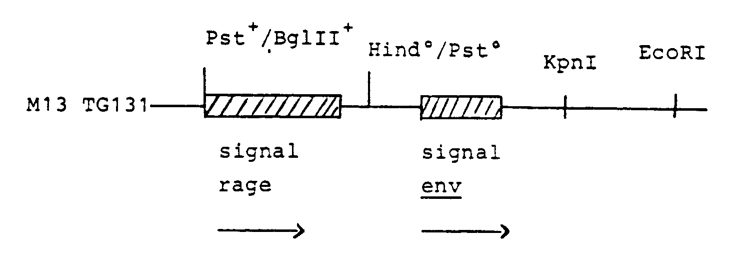
Pst⁺/BglII⁺
Hind°/Pst°
KpnI
EcoRI
M13 TG131
signal
rage
signal
env
Une mutagénèse localisée sur M13 TG160 permet de fusionner en phase les séquences env et glycoprotéine rabique (en formant une boucle). Ceci grâce à l'oligonucléotide
5' GACCCACAATTTTCTGTAATAGGGAATTTCCCAAA 3' 
Signal rage
env
Cys Phe Gly Lys Phe Pro Ile Tyr Thr Gln Lys Leu
5' TGT TTT GGG AAA TTC CCT ATT TAC --- ACA GAA AAA TTG 3'
On obtient ainsi le phage M13 TG161.

Le fragment PvuII-KpnI de M13 TG161 est alors cloné dans pTG1126 coupé par EcoRI traité à la Klenow-KpnI (le site PvuII de M13 TG161 provient de M13 dans la région située en amont du polylinker). Ceci conduit au plasmide pTG1129.

Par clonage du fragment PstI-PstI de pTG1129 dans le plasmide pTG186-POLY coupé par PstI, on obtient le plasmide pTG1131.

### EXEMPLE 9

### Préparation du plasmide pTG1132

Par clonage du fragment PstI-PstI de pTG1128 dans le site PstI de M13 TG131, on obtient le plasmide M13 TG162.

On effectue ensuite une mutagénèse localisée grâce à l'oligonucléotide
5' ATTCCCACTGCTTAGTATTCATTCTGCACCACTC 3'
Ceci permet de placer un codon stop à la fin de la gp120. Les séquences obtenues sont les suivantes : 
R E K R
Séquence originale : CAG AGA GAA AAA AGA GCA GTG
site de clivage présumé
de la gp120
N E Y ***
Séquence mutée : CAG AAT GAA TAC TAA GCA GTG
On obtient ainsi le phage M13 TG168.

Par reclonage du fragment PstI de M13 TG168 dans le site de PstI de pTG186-POLY, on obtient le plasmide pTG1132.

### EXEMPLE 10

### Construction du plasmide pTG1133

Par mutagénèse localisée sur M13 TG162 grâce à l'oligonucléotide suivant :
5' ATTCCCACTGCTTGGTGTTCATTCTGCACCACTC 3'
on obtient un bactériophage dans lequel un site potentiel de de clivage séparant gp 120 et gp 40 a été détruit.

Les séquences modifiées sont les suivantes : 
séquence originale : CAG AGA GAA AAA AGA GCA GTG
R E K R A V
site de clivage
séquence mutée : CAG AAT GAA CAC CAA GCA
N E H Q
On obtient ainsi le phage M13TG165.

Par reclonage du fragment PstI-PstI de M13 TG165 dans pTG186-POLY au site PstI, on obtient le plasmide pTG1133.

### EXEMPLE 11

### Construction du plasmide pTG1134

Par clonage du fragment PstI-PstI de pTG1131 dans le site PstI de M13 TG131, on obtient le phage M13 TG163.

On effectue une mutagénèse localisée sur M13 TG163 afin de détruire le même site de clivage de la gp120 que précédemment. Pour ce faire, on utilise un oligonucléotide :
5' ATTCCCACTGCTTGATGTTCATTCTGCACCACTC 3'
Ceci permet de modifier les séquences de la façon suivante : 
R E K R
Séquence originale : CAG AGA GAA AAA AGA ↑ GCA GTG
Séquence mutée : CAG AAT GAA CAT CAA GCA
N E H Q
On obtient dans ces conditions le phage M13 TG166.

Par reclonage du fragment PstI-PstI de ce phage M13 TG166 dans le site PstI de pTG186-POLY, on obtient le plasmide pTG1134.

### EXEMPLE 12

### Immunoprécipitation des protéines synthétisées par les virus recombinants VV.TG. eLAV.

En opérant comme cela a été décrit précédemment pour le plasmide pTG1125, on obtient les vecteurs vaccine hybrides correspondant aux différents plasmides préparés précédemment.

Ces vecteurs viraux seront appelés respectivement
VV.TG. eLAV 1128
VV.TG. eLAV 1130
VV.TG. eLAV 1131
VV.TG. eLAV 1132
VV.TG. eLAV 1133
VV.TG. eLAV 1134.

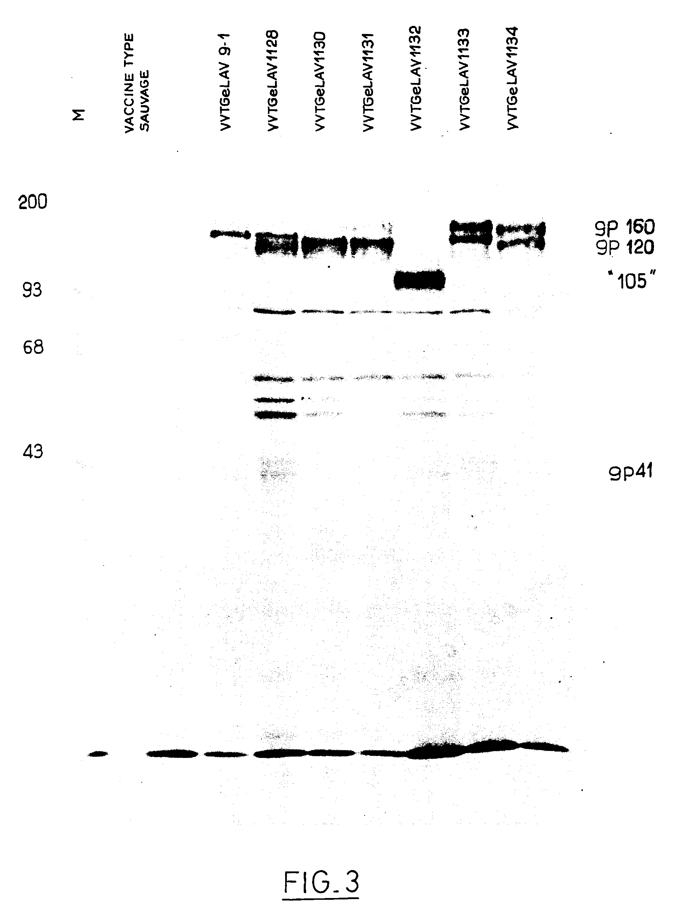

Les protéines obtenues comme cela a été décrit précédemment sont testées par immuno-précipitation (figure 3).

L'ensemble des immuno-précipités fait apparaître pour le virus 9-1, une immuno-précipitation correspondant à la gp160, la gp120 et la gp41.

Il en va de même pour le virus 1128.

Le virus 1130 montre également une gp160 et une gp120.

La protéine correspondant à la gp41 a un poids légèrement inférieur dû à la modification de son extrémité C-terminale.

Le virus 1131 présente un spectre sensiblement identique à celui obtenu pour le virus 1130.

Le virus 1132 ne présente pas bien entendu de protéine correspondant à la gp41. La protéine de 105 kDA présente dans les culots est une isoforme de la gp120 (glycosylation différente).

En ce qui concerne le virus 1133, celui-ci présente bien des protéines 160, 120 et 41 mais les bandes correspondant aux protéines 120 et 41 sont plus faibles que dans les autres spectres.

Il en va de même pour le virus 1134 avec lequel la gp41 présente également un poids moléculaire plus faible mais pour lequel il est clair que le clivage s'est fait avec une cinétique plus lente que celle des virus VV.TG.1125 (9-1) à 1131.

### Exemple 13 Construction du plasmide pTGll35.

Les cinétiques de relargage effectuées sur le virus VV.TG.eLAVll33 et ll34 montrent que, bien que la cinétique de coupure entre la gpl20 et la gp40 soit plus lente, le clivage a encore lieu. L'examen de la séquence d'ADN du gène env révèle un autre site potentiel de clivage (KRR), 8 acides aminés en aval du premier site de clivage.Il peut donc paraître important de muter ce deuxième site afin d'obtenir un virus de la vaccine recombinant qui n'exprime que de la gpl60.

Par mutagénèse localisée sur Ml3TGl66 grâce à l'oligonucléotide suivant :
5'ATTCTGCACCACGTGATTCTGTGCCTTGGTGGGT 3'
on obtient un phage dans lequel le deuxième site de clivage est modifié. Les séquences modifiées sont les suivantes : 
séquence originale : GCA AAG AGA AGA GTG
A K R R V
↑
site de clivage potentiel

séquence mutée : GCA CAG AAT CAC GTG
A Q N H V
Le fragment PstI-PstI du phage obtenu (Ml3TGl8l) est cloné dans pTGl86POLY au site PstI pour générer le plasmide pTGll35.

### Exemple 14 Construction du plasmide pTGll39.

La partie C-terminale du gène env synthétisée par le vecteur de la vaccine recombinant VV.TG.eLAVll35 est une séquence dérivant de la glycoprotéine rabique. Il peut donc paraître utile de disposer également d'un autre recombinant où cette partie C-terminale serait remplacée par la partie C-terminale du gène env du virus LAV.

Pour ce faire, on effectue sur le phage Ml3TGl65 la même mutagénèse que celle effectuée (voir exemple l3) sur le phage Ml3TGl66, pour générer le phage Ml3TGl84.

Le fragment PstI-PstI de Ml3TGl84 est ensuite recloné dans le plasmide pTGl86POLY pour générer le plasmide pTGll39.

### Exemple 15 Construction du plasmide pTGll36.

On voudrait encore disposer d'un virus de la vaccine recombinant exprimant la gpl20 seule. Cette gpl20 sera, contrairement au cas obtenu avec le virus VV.TG.eLAVll32, munie d'une zone d'ancrage C-terminale.

Pour ce faire, on élimine par mutagénèse localisée les séquences correspondant à la gp40 dans le phage Ml3TGl8l à l'aide de l'oligonucléotide suivant :
5'TGCACTCAGTAATACATACACGTGATTCTGTGCCTT 3'
Cet oligonucléotide permet de fusionner en phase les séquences gpl20 (avec les 2 sites de clivage modifiés) et les séquences de la zone transmembranaire de la glycoprotéine rabique 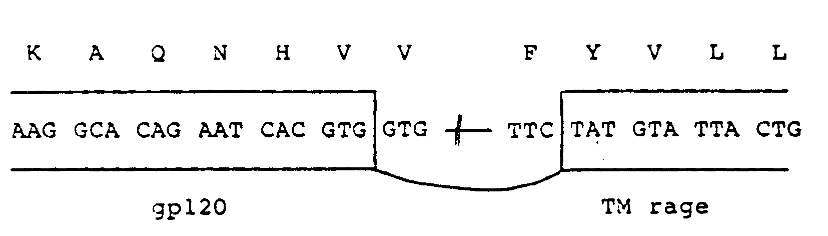
K A Q N H V V F Y V L L
AAG GCA CAG AAT CAC GTG GTG TTC TAT GTA TTA CTG
gp120 TM rage
On obtient ainsi le phage Ml3TGl82. Le fragment PstI-PstI de Ml3TGl82 est ensuite inséré au site PstI de pTGl86POLY pour générer le plasmide pTGll36.

### Exemple 16 Construction du plasmide pTGll37.

Le rôle de la zone hydrophobe située à la partie N-terminale de la gp40 est peu connu. Cette zone de la protéine env pourrait être responsable du pouvoir d'induction de formation des syncitia.

Il semble donc intéressant de produire une gpl60 qui ne contiendrait pas cette séquence.

Pour ce faire, on fusionne en phase les séquences en amont et en aval de la partie codant pour ce peptide hydrophobe dans le phage Ml3TGl8l grâce à l'oligonucléotide suivant :
5'CAATAATTGTCTGGCCTGCACGTGATTCTGTGCCTT 3'
Ceci permet d'obtenir le phage Ml3TGl83 en réalisant la fusion : 
AAG GCA CAG AAT CAC GTG GTG ‡ GTA CAG GCC AGA CAA
K A Q N H V V V Q A R Q
gp120
partie hydrophile
de la gp40
Le fragment PstI-PstI de Ml3TGl83 est recloné dans le site PstI de pTGl86POLY pour générer le plasmide pTGll37.

### Exemple 17 Construction du plasmide pTGll38.

En plus de virus recombinants exprimant la gpl60 ou la gpl20, il peut être utile de générer un virus de la vaccine recombinant exprimant la gp40 seule.

Pour ce faire, on fusionne les séquences codant pour le peptide signal avec les séquences codantes de la gp40 sur le phage Ml3TGl63 grâce à l'oligonucléotide suivant :
5'CAATAATTGTCTGGCCTGAATAGGGAATTTCCCAAA 3'
Ceci permet de générer le phage Ml3TGl80, qui comporte la fusion : 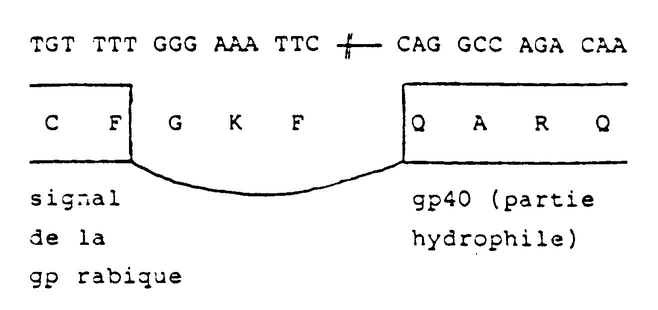
TGT TTT GGG AAA TTC CAG GCC AGA CAA
C F G K F Q A R Q
signal
de la
gp rabique
gp40 (partie
hydrophile)
Le fragment PstI-PstI de Ml3TGl80 est inséré au site PstI de pTGl86POLY pour donner le plasmide pTGll38.

### Exemple 18 Construction du plasmide pTGll62.

Comme dans le cas de la gpl60 (plasmide pTGll39), il peut être important de disposer également d'un virus recombinant exprimant une gp40 dans laquelle la zone d'ancrage et la zone intracytoplasmique soient les séquences du gène env du virus LAV plutôt que les séquences correspondantes de la glycoprotéine rabique.

Pour obtenir ceci, on remplace le fragment HindIII-BglI de Ml3TGl80 par le fragment HindIII-BglI de Ml3TGl65, en générant le phage Ml3TGl90.

Le plasmide pTGll62 est obtenu par clonage du fragment PstI-PstI du phage Ml3TGl90 dans le site PstI du plasmide pTGl86POLY.

### Exemple 19 Construction du plasmide pTGll63.

Il semble aussi important d'obtenir un virus de la vaccine recombinant qui synthétise une gpl60 non clivée et sécrétée dans le milieu. En effet, cette protéine pourrait être utilisée comme vaccin tué, en association avec des adjuvants, ou incluse dans des liposomes ou des ISCOMS (Morein et al., Nature (1984) 308, 5958 p.457-60).

On construit pour cela le bactériophage Ml3TGl94, dans lequel les séquences codant pour les régions extracytoplasmique et intracytoplasmique sont fusionnées en phase, dans le bactériophage M13TG184 grâce à l'oligonucléotide suivant :
5'TCCCTGCCTAACTCTATTTTTTATATACCACAGCCA 3'
Le fragment PstI-PstI de Ml3TGl94 est ensuite cloné au site PstI de pTGl86POLY pour donner pTGll63.

Les protéines recombinantes ainsi obtenues, et en particulier la gp160 non clivable, peuvent être utilisées dans des kits de diagnostic pour détecter les anticorps potentiels présents dans le sang des malades ayant été en contact avec le virus. Ces tests peuvent être mis en oeuvre selon des processus connus de l'homme de l'art, par exemple par ELISA, RIPA, "Westhern Blot" (Immuno-empreinte).

Ces protéines peuvent également être utilisées pour la réalisation d'hybridomes et d'anticorps monoclonaux destinés à détecter la présence de virus dans des échantillons.

Les différents plasmides et phages M13 sont décrits notamment dans les demandes de brevet suivants :
M13 TG131 : Kieny et al., 1983
M13 TGRG151 : WO 83/O4O52
pTG155 PRO : FR 84 O6499
M13 TG13O : Kieny et al., 1983.

Les plasmides suivants ont été déposés le 16 novembre 1984 à la Collection Nationale de Cultures de Microorganismes de l'Institut Pasteur et sont décrits dans le brevet GB-A-84 29O99 :
PJ 19-6 : CNCM N^{o} 366-I
PJ 19-13 : CNCM N^{o} 367-I.

Le plasmide pTG1125 a été déposé le 6 juin 1986 dans la même collection, sous forme de bactérie transformée :
E. coli 1106/pTG1125, sous le n^{o} I-557.

### REFERENCES

1. - Drillien, R., Spehner, D. (1983) Virology 131, 385-393.
2. - Kieny, M.P., Lathe, R. and Lecocq, J.P. 1983. New versatile cloning and sequencing vectors based on bacteriophage M13. Gene 26 : 91-99.
3. - Kieny, M.P., Lathe, R., Drillien, R., Spehner, D., Skory, S., Schmitt, D., Wiktor, T., Koprowski, H. and Lecocq, J.P. 1984. Expression of rabies virus glycoprotein from a recombinant vaccinia virus. Nature 312 : 163-166.
4. - Lathe, R., Hirth, P., Dewilde, M., Harford, N. and Lecocq, J.P. 1980. Cell-free synthesis of biologically active heat-stable enterotoxin of Escherichia coli from a cloned gene. nature 284 : 473-474.
5. - Lathe, R., Kieney, M.P., Schmitt, D., Curtis, P., Lecocq, J.P. (1984) J. Mol. Appl. Genet., vol. 2, 331-342.
6. - Lathe, R., Kieny, M.P., Skory, S. and Lecocq, J.P. (1984) DNA, vol. 3, 173-182.
7. - Lusky, M., Botchan, M. (1981) Nature 293, 79-81.
8. - Mackett, M., Smith, G.L. and Moss, B. 1982. Vaccinia virus : a selectable eukaryotic cloning and expression vector. Proc. Natl. Acad. Sci. USA. 79 : 7415-7419.
9. - Maniatis, T., Fritsch, E.F., Sambrook, J. 1982. Molecular cloning : a laboratory manual. Cold Spring Harbor Lab, N.Y.
10. - Muesing, M.A., Smith, D.H., Cabradilla, C.D., Benton, C.V., Lasky, L.A. and Capon, D.J. 1985. Nucleic acid structure and expression of the human AIDS/lymphadenopathy retrovirus. Nature 313 : 450-458.
11. - Messing et Vieras, Gene 19, 1982, p. 269-276.
12. - Panicali, D. and Paoletti, E. 1982. Construction of poxviruses as cloning vectors : Insertion of the thymidine kinase gene from herpes simplex virus into the DNA of infectious vaccinia virus. Proc. Natl. Acad. Sci. USA. 79 : 4927-4931.
13. - Panicali, D., Davis, S.W., Weinberg, R.L., Paoletti, E. (1983) Proc. Natl. Acad. Sci. USA 80, 5364-5368.
14. - Ratner, L., Haseltine, W., Patarca, R., Livak, K.J., Starcich, B., Josephs, S.F., Doran, E.R., Rafalski, J.A., Whitehorn, E.A., Baumeister, K., Ivanoff, L., Petterway Jr., S.R., Pearson, M.L., Lautenberger, J.A., Papas, T.S., Ghrayeb, J., Chang, N.T., Gallo, R.C. and Wong-Staal, F. Complete nucleotide sequence of the AIDS virus, HTLV-III. 1985. Nature 313 : 277-284.
15. - Sanchez-Pescador et al., 1985. Science 227 : 484-492.
16. - Smith, G.L., Mackett, M., Moss, V. (1983) Nature 302, 490-495.
17. - Smith, G.L., Murphy, B.R., Moss, B. (1983) Proc. Natl. Acad. Sci. USA 80, 7155-7159.
18. - Venkatesan, S., Baroudy, B.M., Moss, B. (1981) Cell 125, 805-813.
19. - Wain-Hobson, S., Sonigo, P., Danos, O., Cole, S. and Alizon, M. Nucleotide Sequence of the AIDS virus, LAV. 1985. Cell 40 : 9-17.
2O. - Weir, J.P., Moss, B. (1983) J. Virol. 46, 530-537.
21. - Zoller, M.J. and Smith, M. 1983. Oligonucleotide-directed mutagenesis of DNA fragments cloned into M13 vectors. In : Methods in Enzymology (Wu, Grossman, Moldave, eds.) 100 : 468-500.

## Revendications (Revendications pour l'(les) Etat(s) contractant(s) suivant(s): AT, BE, CH, DE, FR, GB, GR, IT, LI, LU, NL, SE)

1. Vecteur viral caractérisé en ce qu'il comporte au moins :
- une partie du génome d'un virus,
- une séquence codant pour la séquence signal du précurseur de la glycoprotéine gp160 de l'enveloppe du virus responsable du SIDA, ou une séquence signal provenant d'un virus hétérologue,
- la séquence du gène codant pour la glycoprotéine gp160 de l'enveloppe du virus responsable du SIDA, le gène codant pour ladite gp160 ne comportant pas le site de clivage par les protéases de séquence REKR,
- ainsi que les éléments assurant l'expression de cette glycoprotéine dans des cellules.

2. Vecteur viral selon la revendication 1, caractérisé en ce que le gène de la gp160 code pour une gp160 ne comportant pas les sites de clivages par les protéases de séquence REKR et KRR.

3. Vecteur viral selon la revendication 1, caractérisé en ce que la séquence REKR est remplacée par la séquence NEHQ.

4. Vecteur viral selon la revendication 2, caractérisé en ce que les séquences REKR et KRR sont remplacées respectivement par les séquences NEHQ et QNH.

5. Vecteur viral selon l'une des revendications 1 à 4, caractérisé en ce que la partie du génome d'un virus est une partie du génome d'un poxvirus.

6. Vecteur viral selon la revendication 5, caractérisé en ce que le poxvirus est le virus de la vaccine.

7. Vecteur viral selon les revendications 1 à 6, caractérisé en ce qu'il ne comporte pas la séquence codant pour la région transmembranaire de la gp160 naturelle.

8. Vecteur viral selon les revendications 1 à 7, caractérisé en ce que la séquence codant pour le peptide hydrophobe C-terminal correspondant à la zone transmembranaire (tm) de la gp160 naturelle a été mutée pour remplacer le codon Arg par un codon Ile.

9. Vecteur viral selon la revendication 7, caractérisé en ce qu'il comporte une séquence codant pour une région transmembranaire provenant d'un virus hétérologue, notamment du virus de la rage.

10. Vecteur viral selon la revendication 7, caractérisé en ce qu'il ne comp̂orte p̂as de séquence codant pour une région d'ancrage transmembranaire.

11. Vecteur viral selon l'une des revendications 1 à 10, caractérisé en ce que la séquence d'ADN codant pour la gp160 est sous la dépendance d'un promoteur d'un gène d'un poxvirus.

12. Vecteur viroal selon la revendication 10, caractérisé en ce que le promoteur est un promoteur d'un gène du virus de la vaccine.

13. Vecteur viral selon la revendication 12, caractérisé en ce que la séquence d'ADN codant pour la gp160 est sous le contrôle du promoteur du gène de la protéine 7,5 K du virus de la vaccine.

14. Vecteur viral selon l'une des revendications 11 à 13, caractérisé en ce que la séquence codant pour la pg160 est clonée dns le gène TK du virus de la vaccine.

15. Vecteur viral selon l'une des revendications 1 à 7 et 9 à 14, caractérisé en ce que le peptide hydrophobe C-terminal de la gp160 est délété.

16. ADN recombinant correspondant à un vecteur viral selon l'une des revendications 1 à 15.

17. Culture de cellules de mammifères infectées par un vecteur viral selon l'une des revendications 1 à 15 ou comportant un ADN selon la revendication 16.

18. Procédé de préparation de glycoprotéines d'enveloppe de virus responsable du SIDA, caractérisé en ce que l'on cultive des cellules selon la revendication 17 et que l'on récupère la glycoprotéine produite.

19. Glycoprotéine d'enveloppe gp 160 non clivable du virus responsable du SIDA que l'on peut obtenir notamment par mise en oeuvre du procédé selon la revendication 18.

20. Glycoprotéine gp160 non clivable de l'enveloppe du virus responsable du SIDA caractérisée en ce qu'elle ne comporte pas le site de clivage par les protéases de séquence REKR de la glycoprotéine gp160 naturelle.

21. Glycoprotéine gp160 non clivable selon la revendication 20, caractérisée en ce qu'elle ne comporte pas les sites de clivage par les protéases de séquences REKR et KRR de la glycoprotéine gp160 naturelle.

22. Glycoprotéine gp160 non clivable selon la revendication 20 ou 21, caractérisée en ce que la séquence REKR est remplacée par la séquence NEHQ.

23. Glycoprotéine gp160 non clivable selon la revendication 20 ou 21, caractérisée en ce que les séquences REKR et KRR de la gp160 naturelle sont remplacées par les séquences NEHQ et QNH respectivement.

24. Glycoprotéine selon l'une des revendications 20 à 23 caractérisée en ce qu'elle ne comporte pas la région d'ancrage transmembranaire de la gp160 naturelle.

25. Glycoprotéine selon la revendication 24 caractérisé en ce qu'elle comporte la région transmembranaire du virus de la rage.

26. Glycoprotéine selon la revendication 24 caractérisé en ce que le peptide hydrophobe C-terminal correspondant à la région transmembranaire de la gp160 est modifié , l'arginine étant remplacée par une isoleucine.

27. Glycoprotéine selon la revendication 24 caractérisé en ce qu'elle ne comporte pas de région d'ancrage transmembranaire.

28. Glycoprotéine selon l'une des revendications 22 à 25 ou 27 caractérisé en ce que le peptide hydrophobe C-terminal de la gp160 naturelle est délété.

29. Vaccin caractérisé en ce qu'il est constitué par un vecteur viral selon l'une des revendications 1 à 13 et/ou une glycoprotéine selon l'une des revendications 19 ou 28.

30. Anticorps dressés contre les glycoprotéines d'enveloppe du virus responsable du SIDA, selon les revendications 19 à 28 autres que les anticorps dressés contre les glycoprotéines gp160 naturelles.

## Revendications (Revendications pour l'(les) Etat(s) contractant(s) suivant(s): ES)

1. Procédé de préparation d'un vecteur viral caractérisé en ce qu'on insère dans une partie du génome d'un virus:
- une séquence codant pour la séquence signal du précurseur de la glycoprotéine gp160 de l'enveloppe du virus responsable du SIDA, ou une séquence signal provenant d'un virus hétérologue,
- la séquence du gène codant pour la glycoprotéine gp160 de l'enveloppe du virus responsable du SIDA, le gène codant pour ladite gp160 ne comportant pas le site de clivage par les protéases de séquence REKR,
- ainsi que les éléments assurant l'expression de cette glycoprotéine dans des cellules.

2. Procédé de préparation selon la revendication 1, caractérisé en ce que le gène de la gp160 code pour une gp160 ne comportant pas les sites de clivages par les protéases de séquence REKR et KRR.

3. Procédé de préparation selon la revendication 1, caractérisé en ce que la séquence REKR est remplacée par la séquence NEHQ.

4. Procédé de préparation selon la revendication 2, caractérisé en ce que les séquences REKR et KRR sont remplacées respectivement par les séquences NEHQ et QNH.

5. Procédé de préparation selon l'une des revendications 1 à 4, caractérisé en ce que la partie du génome d'un virus est une partie du génome d'un poxvirus.

6. Procédé de préparation selon la revendication 5, caractérisé en ce que le poxvirus est le virus de la vaccine.

7. Procédé de préparation selon les revendications 1 à 6, caractérisé en ce qu'il ne comporte pas la séquence codant pour la région transmembranaire de la gp160 naturelle.

8. Procédé de préparation selon les revendications 1 à 7, caractérisé en ce que la séquence codant pour le peptide hydrophobe C-terminal correspondant à la zone transmembranaire (tm) de la gp160 naturelle a été mutée pour remplacer le codon Arg par un codon Ile.

9. Procédé de préparation selon la revendication 7, caractérisé en ce qu'il comporte une séquence codant pour une région transmembranaire provenant d'un virus hétérologue, notamment du virus de la rage.

10. Procédé de préparation selon la revendication 7, caractérisé en ce qu'il ne comporte pas de séquence codant pour une région d'ancrage transmembranaire.

11. Procédé de préparation selon l'une des revendications 1 à 10, caractérisé en ce que la séquence d'ADN codant pour la gp160 est sous la dépendance d'un promoteur d'un gène d'un poxvirus.

12. Procédé de préparation selon la revendication 10, caractérisé en ce que le promoteur est un promoteur d'un gène du virus de la vaccine.

13. Procédé de préparation selon la revendication 12, caractérisé en ce que la séquence d'ADN codant pour la gp160 est sous le contrôle du promoteur du gène de la protéine 7,5 K du virus de la vaccine.

14. Procédé de préparation selon l'une des revendications 11 à 13, caractérisé en ce que la séquence codant pour la gp160 est clonée dans le gène TK du virus de la vaccine.

15. Procédé de préparation selon l'une des revendications 1 à 7 et 9 à 14, caractérisé en ce que le peptide hydrophobe C-terminal de la gp160 est délété.

16. Procédé de préparation de glycoprotéines d'enveloppe de virus responsable du SIDA, caractérisé en ce que l'on cultive des cellules de mammifères infectées par un vecteur viral selon l'une des revendications 1 à 15 ou comportant un ADN recombinant correspondant audit vecteur viral et que l'on récupère la glycoprotéine produite.

17. Procédé de préparation selon la revendication 16 caractérisé en ce qu'on prépare la glycoprotéine d'enveloppe gp160 non clivable du virus responsable du SIDA.

18. Procédé de préparation selon la revendication 17 caractérisé en ce que la glycoprotéine gp160 non clivable de l'enveloppe du virus responsable du SIDA ne comporte pas le site de clivage par les protéases de séquence REKR de la glycoprotéine gp160 naturelle.

19. Procédé de préparation selon la revendication 18 caractérisé en ce que la glycoprotéine gp160 non clivable ne comporte pas les sites de clivage par les protéases de séquences REKR et KRR de la glycoprotéine gp160 naturelle.

20. Procédé de préparation selon la revendication 18 ou 19 caractérisé en ce que, dans la glycoprotéine gp160 non clivable, la séquence REKR est remplacée par la séquence NEHQ.

21. Procédé de préparation selon la revendication 18 ou 19, caractérisé en ce que les séquences REKR et KRR de la gp160 naturelle sont remplacées par les séquences NEHQ et QNH respectivement.

22. Procédé de préparation selon l'une des revendications 18 à 21 caractérisé en ce qu'il ne comporte pas la région d'ancrage transmembranaire de la gp160 naturelle.

23. Procédé de préparation selon la revendication 22 caractérisé en ce que la glycoprotéine comporte la région transmembranaire du virus de la rage.

24. Procédé de préparation selon la revendication 22 caractérisé en ce que le peptide hydrophobe C-terminal correspondant à la région transmembranaire de la gp160 est modifié, l'arginine étant remplacée par une isoleucine.

25. Procédé de préparation selon la revendication 22 caractérisé en ce que la glycoprotéine ne comporte pas de région d'ancrage transmembranaire.

26. Procédé de préparation selon la revendication 22 ou 23 caractérisé en ce que le peptide hydrophobe C-terminal de la gp160 naturelle est délété.

27. Procédé de préparation d'un anticorps caractérisé en ce qu'il est obtenu à partir d'une glycoprotéine d'enveloppe du virus responsable du SIDA, selon les revendications 16 à 26, autres que les anticorps dressés contre les glycoprotéines gp160 naturelles.

## Claims (Claims for the following Contracting State(s): AT, BE, CH, DE, FR, GB, GR, IT, LI, LU, NL, SE)

1. Viral vector, characterized in that it contains at least:
- a part of the genome of a virus,
- a sequence coding for the signal sequence of the precursor of the glycoprotein gp160 of the envelope of the virus responsible for AIDS, or a signal sequence from a heterologous virus,
- the sequence of the gene coding for the glycoprotein gp160 of the envelope of the virus responsible for AIDS, the gene coding for the said gp160 not containing the site of cleavage by the proteases of sequence REKR,
- and also the elements ensuring the expression of this glycoprotein in cells.

2. Viral vector according to Claim 1, characterized in that the gene of the gp160 codes for a gp160 not containing the sites of cleavage by the proteases of sequence REKR and KRR.

3. Viral vector according to Claim 1, characterized in that the REKR sequence is replaced by the NEHQ sequence.

4. Viral vector according to Claim 2, characterized in that the REKR and KRR sequences are replaced respectively by the NEHQ and QNH sequences.

5. Viral vector according to one of Claims 1 to 4, characterized in that the part of the genome of a virus is a part of the genome of a pox virus.

6. Viral vector according to Claim 5, characterized in that the pox virus is the virus of the vaccine.

7. Viral vector according to Claims 1 to 6, characterized in that it does not contain the sequence coding for the transmembrane region of the natural gp160.

8. Viral vector according to Claims 1 to 7, characterized in that the sequence coding for the C-terminal hydrophobic peptide corresponding to the transmembrane (tm) zone of the natural gp160 has been mutated to replace the Arg codon by an Ile codon.

9. Viral vector according to Claim 7, characterized in that it contains a sequence coding for a transmembrane region from a heterologous virus, especially from the rabies virus.

10. Viral vector according to Claim 7, characterized in that it does not contain a sequence coding for a transmembrane anchorage region.

11. Viral vector according to one of Claims 1 to 10, characterized in that the DNA sequence coding for the gp160 is under the dependence of a promoter of a pox virus gene.

12. Viral vector according to Claim 10, characterized in that the promoter is a promoter of a gene of the vaccine virus.

13. Viral vector according to Claim 12, characterized in that the DNA sequence coding for the gp160 is under the control of the promoter of the gene of the 7.5 K protein of the vaccine virus.

14. Viral vector according to one of Claims 11 to 13, characterized in that the sequence coding for the gp160 is cloned in the TK gene of the vaccine virus.

15. Viral vector according to one of Claims 1 to 7 and 9 to 14, characterized in that the C-terminal hydrophobic peptide of the gp160 is deleted.

16. Recombinant DNA corresponding to a viral vector according to one of Claims 1 to 15.

17. Culture of mammalian cells infected by a viral vector according to one of Claims 1 to 15 or containing a DNA according to Claim 16.

18. Method of preparation of envelope glycoproteins of the virus responsible for AIDS, characterized in that cells according to Claim 17 are cultured and in that the glycoprotein produced is recovered.

19. Non-cleavable gp160 envelope glycoprotein of the virus responsible for AIDS which can be obtained especially by employing the process according to Claim 18.

20. Non-cleavable gp160 glycoprotein of the envelope of the virus responsible for AIDS, characterized in that it does not contain the site of cleavage by the proteases of sequence REKR of the natural gp160 glycoprotein.

21. Non-cleavable gp160 glycoprotein according to Claim 20, characterized in that it does not contain the sites of cleavage by the proteases of sequences REKR and KRR of the natural gp160 glycoprotein.

22. Non-cleavable gp160 glycoprotein according to Claim 20 or 21, characterized in that the REKR sequence is replaced by the NEHQ sequence.

23. Non-cleavable gp160 glycoprotein according to Claim 20 or 21, characterized in that the REKR and KRR sequences of the natural gp160 are replaced by the NEHQ and QNH sequences respectively.

24. Glycoprotein according to one of Claims 20 to 23, characterized in that it does not contain the transmembrane anchorage region of the natural gp160.

25. Glycoprotein according to Claim 24, characterized in that it contains the transmembrane region of the rabies virus.

26. Glycoprotein according to Claim 24, characterized in that the C-terminal hydrophobic peptide corresponding to the transmembrane region of the gp160 is modified, the arginine being replaced by an isoleucine.

27. Glycoprotein according to Claim 24, characterized in that it does not contain any transmembrane anchorage region.

28. Glycoprotein according to one of Claims 22 to 25 or 27, characterized in that the C-terminal hydrophobic peptide of the natural gp160 is deleted.

29. Vaccine, characterized in that it consists of a viral vector according to one of Claims 1 to 13 and/or a glycoprotein according to one of Claims 19 or 28.

30. Antibodies raised against the envelope glycoproteins of the virus responsible for AIDS, according to Claims 19 to 28, other than the antibodies raised against the natural gp160 glycoproteins.

## Claims (Claims for the following Contracting State(s): ES)

1. Method of preparation of a viral vector, characterized in that:
- a sequence coding for the signal sequence of the precursor of the glycoprotein gp160 of the envelope of the virus responsible for AIDS, or a signal sequence from a heterologous virus,
- the sequence of the gene coding for the glycoprotein gp160 of the envelope of the virus responsible for AIDS, the gene coding for the said gp160 not containing the site of cleavage by the proteases of sequence REKR,
- and also the elements ensuring the expression of this glycoprotein in cells,
are inserted into a part of the genome of a virus.

2. Method of preparation according to Claim 1, characterized in that the gene of the gp160 codes for a gp160 not containing the sites of cleavage by the proteases of sequence REKR and KRR.

3. Method of preparation according to Claim 1, characterized in that the REKR sequence is replaced by the NEHQ sequence.

4. Method of preparation according to Claim 2, characterized in that the REKR and KRR sequences are replaced respectively by the NEHQ and QNH sequences.

5. Method of preparation according to one of Claims 1 to 4, characterized in that the part of the genome of a virus is a part of the genome of a pox virus.

6. Method of preparation according to Claim 5, characterized in that the pox virus is the virus of the vaccine.

7. Method of preparation according to Claims 1 to 6, characterized in that it does not contain the sequence coding for the transmembrane region of the natural gp160.

8. Method of preparation according to Claims 1 to 7, characterized in that the sequence coding for the C-terminal hydrophobic peptide corresponding to the transmembrane (tm) zone of the natural gp160 has been mutated to replace the Arg codon by an Ile codon.

9. Method of preparation according to Claim 7, characterized in that it contains a sequence coding for a transmembrane region from a heterologous virus, especially from the rabies virus.

10. Method of preparation according to Claim 7, characterized in that it does not contain a sequence coding for a transmembrane anchorage region.

11. Method of preparation according to one of Claims 1 to 10, characterized in that the DNA sequence coding for the gp160 is under the dependence of a promoter of a pox virus gene.

12. Method of preparation according to Claim 10, characterized in that the promoter is a promoter of a gene of the vaccine virus.

13. Method of preparation according to Claim 12, characterized in that the DNA sequence coding for the gp160 is under the control of the promoter of the gene of the 7.5 K protein of the vaccine virus.

14. Method of preparation according to one of Claims 11 to 13, characterized in that the sequence coding for the gp160 is cloned in the TK gene of the vaccine virus.

15. Method of preparation according to one of Claims 1 to 7 and 9 to 14, characterized in that the C-terminal hydrophobic peptide of the gp160 is deleted.

16. Method of preparation of virus envelope glycoproteins responsible for AIDS, characterized in that cells of mammals infected by a viral vector according to one of Claims 1 to 15 or containing a recombinant DNA corresponding to the said viral vector are cultured and the glycoprotein produced is recovered.

17. Method of preparation according to Claim 16, characterized in that the non-cleavable gp160 envelope glycoprotein of the virus responsible for AIDS is prepared.

18. Method of preparation according to Claim 17, characterized in that the non-cleavable gp160 glycoprotein of the envelope of the virus responsible for AIDS does not contain the site of cleavage by the proteases of sequence REKR of the natural gp160 glycoprotein.

19. Method of preparation according to Claim 18, characterized in that the non-cleavable gp160 glycoprotein does not contain the sites of cleavage by the proteases of sequences REKR and KRR of the natural gp160 glycoprotein.

20. Method of preparation according to Claim 18 or 19, characterized in that, in the non-cleavable gp160 glycoprotein, the REKR sequence is replaced by the NEHQ sequence.

21. Method of preparation according to Claim 18 or 19, characterized in that the REKR and KRR sequences of the natural gp160 are replaced by the NEHQ and QNH sequences respectively.

22. Method of preparation according to one of Claims 18 to 21, characterized in that the glycoprotein does not contain the transmembrane anchorage region of the natural gp160.

23. Method of preparation according to Claim 22, characterized in that the glycoprotein contains the transmembrane region of the rabies virus.

24. Method of preparation according to Claim 22, characterized in that the C-terminal hydrophobic peptide corresponding to the transmembrane region of the gp160 is modified, the arginine being replaced by an isoleucine.

25. Method of preparation according to Claim 22, characterized in that the glycoprotein does not contain the transmembrane anchorage region.

26. Method of preparation according to Claim 22 or 23, characterized in that the C-terminal hydrophobic peptide of the natural gp160 is deleted.

27. Method of preparation of an antibody, characterized in that it is obtained from a virus envelope glycoprotein responsible for AIDS, according to Claims 16 to 26, other than the antibodies raised against the natural gp160 glycoproteins.

## Patentansprüche (Patentansprüche für folgende(n) Vertragsstaat(en): AT, BE, CH, DE, FR, GB, GR, IT, LI, LU, NL, SE)

1. Virus-Vektor, dadurch gekennzeichnet, daß er mindestens umfaßt:
- einen Teil des Genoms eines Virus,
- eine Sequenz, die für die Signal-Sequenz des Vorläufers des Glycoproteins gp160 der Hülle des AIDS-Virus codiert, oder eine Signal-Sequenz, die aus einem heterologen Virus stammt,
- die Sequenz des Gens, das für das Glycoprotein gp160 der Hülle des AIDS-Virus codiert, wobei das für das genannte gp160 codierende Gen nicht die Schnittstelle für die Proteasen mit der Sequenz REKR enthält,
- sowie die Elemente, welche die Expression dieses Glycoproteins in Zellen gewährleistet.

2. Virus-Vektor nach Anspruch 1, dadurch gekennzeichnet, daß das Gen des gp160 für ein gp160 codiert, das nicht die Schnittstellen für die Proteasen mit der Sequenz REKR und KRR enthält.

3. Virus-Vektor nach Anspruch 1, dadurch gekennzeichnet, daß die Sequenz REKR durch die Sequenz NEHQ ersetzt ist.

4. Virus-Vektor nach Anspruch 2, dadurch gekennzeichnet, daß die Sequenzen REKR und KRR jeweils durch die Sequenzen NEHQ und QNH ersetzt sind.

5. Virus-Vektor nach einem der Ansprüche 1 bis 4, dadurch gekennzeichnet, daß der Teil des Genoms eines Virus ein Teil des Genoms eines Pocken-Virus ist.

6. Virus-Vektor nach Anspruch 5, dadurch gekennzeichnet, daß das Pocken-Virus das Vakzine-Virus ist.

7. Virus-Vektor nach den Ansprüchen 1 bis 6, dadurch gekennzeichnet, daß er nicht die für den Transmembran-Bereich des natürlichen gp160 codierende Sequenz enthält.

8. Virus-Vektor nach den Ansprüchen 1 bis 7, dadurch gekennzeichnet, daß die für das C-terminale hydrophobe Peptid codierende Sequenz, die der Transmembran(tm)-Zone des natürlichen gp160 entspricht, durch Ersatz des Codons Arg durch einen Codon Ile mutiert worden ist.

9. Virus-Vektor nach Anspruch 7, dadurch gekennzeichnet, daß er eine Sequenz aufweist, die für einen Transmembran-Bereich codiert, die aus einem heterologen Virus, insbesondere dem Tollwut-Virus, stammt.

10. Virus-Vektor nach Anspruch 7, dadurch gekennzeichnet, daß er keine Sequenz aufweist, die für einen Transmembran-Verankerungsbereich codiert.

11. Virus-Vektor nach einem der Ansprüche 1 bis 10, dadurch gekennzeichnet, daß die DNA-Sequenz, die für das gp160 codiert, abhängig ist von einem Promotor eines Pocken-Virus-Gens.

12. Virus-Vektor nach Anspruch 10, dadurch gekennzeichnet, daß der Promotor ein Promotor eines Gens des Vakzine-Virus ist.

13. Virus-Vektor nach Anspruch 12, dadurch gekennzeichnet, daß die DNA-Sequenz, die für das gp160 codiert, unter der Kontrolle des Promotors des Gens des Proteins 7,5 K des Vakzine-Virus steht.

14. Virus-Vektor nach einem der Ansprüche 11 bis 13, dadurch gekennzeichnet, daß die für das gp160 codierende Sequenz in das TK-Gen des Vakzine-Virus cloniert ist.

15. Virus-Vektor nach einem der Ansprüche 1 bis 7 und 9 bis 14, dadurch gekennzeichnet, daß das C-terminale hydrophobe Peptid des gp160 deletiert ist.

16. Rekombinante DNA, die einem Virus-Vektor nach einem der Ansprüche 1 bis 15 entspricht.

17. Kultur von Säugetier-Zellen, die mit einem Virus-Vektor nach einem der Ansprüche 1 bis 15 infiziert sind oder eine DNA nach Anspruch 16 enthalten.

18. Verfahren zur Herstellung von Hüllen-Glycoproteinen des AIDS-Virus, dadurch gekennzeichnet, daß man Zellen gemäß Anspruch 17 kultiviert und das gebildete Glycoprotein gewinnt.

19. Nicht-schneidbares Hüllen-Glycoprotein gp160 des AIDS-Virus, das insbesondere durch Durchführung des Verfahrens nach Anspruch 18 erhältlich ist.

20. Nicht-schneidbares Glycoprotein gp160 der Hülle des AIDS-Virus, dadurch gekennzeichnet, daß es keine Schnittstelle für die Proteasen mit der Sequenz REKR des natürlichen Glycoproteins gp160 aufweist.

21. Nicht-schneidbares Glycoprotein gp160 nach Anspruch 20, dadurch gekennzeichnet, daß es keine Schnittstellen für die Proteasen mit den Sequenzen REKR und KRR des natürlichen Glycoproteins gp160 aufweist.

22. Nicht-schneidbares Glycoprotein gp160 nach Anspruch 21, dadurch gekennzeichnet, daß die Sequenz REKR durch die Sequenz NEHQ ersetzt ist.

23. Nicht-schneidbares Glycoprotein gp160 nach Anspruch 20 oder 21, dadurch gekennzeichnet, daß die Sequenzen REKR und KRR des natürlichen gp160 jeweils durch die Sequenzen NEHQ und KNH ersetzt sind.

24. Glycoprotein nach einem der Ansprüche 20 bis 23, dadurch gekennzeichnet, daß es nicht den Transmembran-Verankerungsbereich des natürlichen gp160 aufweist.

25. Glycoprotein nach Anspruch 24, dadurch gekennzeichnet, daß es den Transmembran-Bereich des Tollwut-Virus umfaßt.

26. Glycoprotein nach Anspruch 24, dadurch gekennzeichnet, daß das C-terminale hydrophobe Peptid, das dem Transmembran-Bereich des gp160 entspricht, modifiziert ist, wobei das Arginin durch ein Isoleucin ersetzt ist.

27. Glycoprotein nach Anspruch 24, dadurch gekennzeichnet, daß es keinen Transmembran-Verankerungsbereich aufweist.

28. Glycoprotein nach einem der Ansprüche 22 bis 25 oder 27, dadurch gekennzeichnet, daß das C-terminale hydrophobe Peptid des natürlichen gp160 deletiert ist.

29. Vakzine, dadurch gekennzeichnet, daß es besteht aus einem Virus-Vektor nach einem der Ansprüche 1 bis 13 und/oder einem Glycoprotein nach einem der Ansprüche 19 oder 28.

30. Antikörper, die gegen die Hüllen-Glycoproteine des AIDS-Virus gerichtet sind, nach den Ansprüchen 19 bis 28, ausgenommen die Antikörper, die gegen die natürlichen Glycoproteine gp160 gerichtet sind.

## Patentansprüche (Patentansprüche für folgende(n) Vertragsstaat(en): ES)

1. Verfahren zur Herstellung eines Virus-Vektors, dadurch gekennzeichnet, daß man in einen Teil des Genoms eines Virus inseriert:
- eine Sequenz, die für die Signal-Sequenz des Vorläufers des Glycoproteins gp160 der Hülle des AIDS-Virus codiert, oder eine Signal-Sequenz, die aus einem heterologen Virus stammt,
- die Sequenz des Gens, das für das Glycoprotein gp160 der Hülle des AIDS-Virus codiert, wobei das für das genannte gp160 codierende Gen nicht die Schnittstelle für die Proteasen mit der Sequenz REKR umfaßt,
- sowie die Elemente, welche die Expression dieses Glycoproteins in Zellen gewährleistet.

2. Verfahren nach Anspruch 1, dadurch gekennzeichnet, daß das Gen des gp160 für ein gp160 codiert, das nicht die Schnittstellen für die Proteasen mit der Sequenz REKR und KRR enthält.

3. Verfahren nach Anspruch 1, dadurch gekennzeichnet, daß die Sequenz REKR durch die Sequenz NEHQ ersetzt ist.

4. Verfahren nach Anspruch 2, dadurch gekennzeichnet, daß die Sequenzen REKR und KRR jeweils durch die Sequenzen NEHQ und QNH ersetzt sind.

5. Verfahren nach einem der Ansprüche 1 bis 4, dadurch gekennzeichnet, daß der Teil des Genoms eines Virus ein Teil des Genoms eines Pocken-Virus ist.

6. Verfahren nach Anspruch 5, dadurch gekennzeichnet, daß das Pocken-Virus das Vakzine-Virus ist.

7. Verfahren nach einem der Ansprüche 1 bis 6, dadurch gekennzeichnet, daß er keine die für den Transmembranbereich des natürlichen gp160 codierende Sequenz enthält.

8. Verfahren nach den Ansprüchen 1 bis 7, dadurch gekennzeichnet, daß die für das C-terminale hydrophobe Peptid codierende Sequenz, die der Transmembran(tm)-Zone des natürlichen gp160 entspricht, durch Ersatz des Codons Arg durch einen Codon Ile mutiert worden ist.

9. Verfahren nach Anspruch 7, dadurch gekennzeichnet, daß er eine Sequenz aufweist, die für einen Transmembran-Bereich codiert, die aus einem heterologen Virus, insbesondere dem Tollwut-Virus, stammt.

10. Verfahren nach Anspruch 7, dadurch gekennzeichnet, daß er keine Sequenz aufweist, die für einen Transmembran-Verankerungsbereich codiert.

11. Verfahren nach einem der Ansprüche 1 bis 10, dadurch gekennzeichnet, daß die DNA-Sequenz, die für das gp160 codiert, abhängig ist von einem Promotor eines Pocken-Virus-Gens.

12. Verfahren nach Anspruch 10, dadurch gekennzeichnet, daß der Promotor ein Promotor eines Gens des Vakzine-Virus ist.

13. Verfahren nach Anspruch 12, dadurch gekennzeichnet, daß die DNA-Sequenz, die für das gp160 codiert, unter der Kontrolle des Promotors des Gens des Proteins 7,5 K des Vakzine-Virus steht.

14. Verfahren nach einem der Ansprüche 11 bis 13, dadurch gekennzeichnet, daß die für das gp160 codierende Sequenz in das TK-Gen des Vakzine-Virus cloniert ist.

15. Verfahren nach einem der Ansprüche 1 bis 7 und 9 bis 14, dadurch gekennzeichnet, daß das C-terminale hydrophobe Peptid des gp160 deletiert ist.

16. Verfahren zur Herstellung von Hüllen-Glycoproteinen des AIDS-Virus, dadurch gekennzeichnet, daß man Säugetier-Zellen, die durch einen Virus-Vektor nach einem der Ansprüche 1 bis 15 infiziert sind oder eine dem genannten Virus-Vektor entsprechende rekombinante DNA aufweisen, kultiviert und daß man das gebildete Glycoprotein gewinnt.

17. Verfahren nach Anspruch 16, dadurch gekennzeichnet, daß man das nicht-schneidbare Hüllen-Glycoprotein gp160 des AIDS-Virus herstellt.

18. Verfahren nach Anspruch 17, dadurch gekennzeichnet, daß das nicht-schneidbare Glycoprotein gp160 der Hülle des AIDS-Virus keine Schnittstelle für die Proteasen mit der Sequenz REKR des natürlichen Glycoproteins gp160 aufweist.

19. Verfahren nach Anspruch 18, dadurch gekennzeichnet, daß das nicht-schneidbare Glycoprotein gp160 keine Schnittstellen für die Proteasen mit den Sequenzen REKR und KRR des natürlichen Glycoproteins gp160 aufweist.

20. Verfahren nach Anspruch 18 oder 19, dadurch gekennzeichnet, daß in dem nicht-schneidbaren Glycoprotein gp160 die Sequenz REKR durch die Sequenz NEHQ ersetzt ist.

21. Verfahren nach Anspruch 18 oder 19, dadurch gekennzeichnet, daß die Sequenzen REKR und KRR des natürlichen gp160 jeweils durch die Sequenzen NEHQ und QNH ersetzt sind.

22. Verfahren nach einem der Ansprüche 18 bis 21, dadurch gekennzeichnet, daß er nicht den Transmembran-Verankerungsbereich des natürlichen gp160 aufweist.

23. Verfahren nach Anspruch 22, dadurch gekennzeichnet, daß das Glycoprotein den Transmembran-Bereich des Tollwut-Virus umfaßt.

24. Verfahren nach Anspruch 22, dadurch gekennzeichnet, daß das C-terminale hydrophobe Peptid, das dem Transmembran-Bereich des gp160 entspricht, modifiziert wird, wobei das Arginin durch ein Isoleucin ersetzt wird.

25. Verfahren nach Anspruch 22, dadurch gekennzeichnet, daß das Glycoprotein keinen Transmembran-Verankerungsbereich aufweist.

26. Verfahren nach Anspruch 22 oder 23, dadurch gekennzeichnet, daß das C-terminale hydrophobe Peptid des natürlichen gp160 deletiert wird.

27. Verfahren zur Herstellung eines Antikörpers, dadurch gekennzeichnet, daß er erhalten wird aus einem Hüllen-Glycoprotein des AIDS-Virus nach den Ansprüchen 16 bis 26, ausgenommen die Antikörper, die gegen die natürlichen Glycoproteine gp160 gerichtet sind.
